# EUROPEAN PATENT APPLICATION

(11) **EP 4 488 262 A1**
(43) Date of publication of application: **08.01.2025**
(21) Application number: 22929644.7
(22) Date of filing: 13.12.2022
(51) Int. Cl.: C07D 209/62, C07D 209/58, C07D 209/56, C07C 311/16, C09K 9/02, G01N 21/25

(54) **1,6-DIYNE COMPOUND AND PREPARATION METHOD THEREFOR, AND PRODUCT OBTAINED USING 1,6-DIYNE COMPOUND AS RAW MATERIAL AND USE THEREOF**

(30) Priority: 04.03.2022 CN 202210209604; 04.03.2022 CN 202210208626; 04.03.2022 CN 202210218223; 04.03.2022 CN 202210209603
(71) Applicant: Fujian Institute Of Research On The Structure Of Matter, Chinese Academy Of Sciences, Fuzhou, Fujian 350002 (CN)
(72) Inventor: BAO, Hongli, Fuzhou, Fujian 350002 (CN); YE, Changqing, Fuzhou, Fujian 350002 (CN); HUANG, Rui, Fujian 350002 (CN); ZHU, Nengbo, Fuzhou, Fujian 350002 (CN)
(74) Representative: Bayramoglu et al.
(86) International application number: PCT/CN2022/138827
(87) International publication number: WO 2023/165217

(57) **Abstract**

A 1,6-diynes compound, its preparation method, and products obtained from 1,6-diynes as raw material and their applications are disclosed, belonging to the field of organic synthesis. The compound represented by a general formula (I), and its tautomers, polymorphs, solvates, or salts thereof, 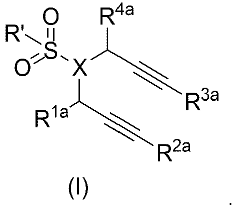 The 1,6-diyne compounds provided in the present application have variable structures, are easy to be synthesized, and are suitable for industrial production; they can be used for the synthesis of a class of benzoisoindole dimer compounds and their derivatives, cell imaging, and detecting reactive oxygen species (such as sodium hypochlorite, hydrogen peroxide, ozone, etc.). The 3-arylbenzoisoindole compound provided in the present application can be used as an important compound and play an important role in pharmaceuticals or fluorescent materials.

## Description

### TECHNICAL FIELD

The present application relates to a 1,6-diynes compound, its preparation method, and products obtained from 1,6-diynes as raw material and their applications, belonging to the field of organic synthesis.

### BACKGROUND TECHNOLOGY

Polycyclic compounds are widely found in natural products, pharmaceuticals, and materials. Due to their large conjugated structures and good luminescent properties, polycyclic compounds have extensive applications in the field of luminescent materials. Therefore, the synthesis methods and application studies of polycyclic compounds have attracted the attention of organic synthesis chemists. Currently, there are two main approaches to synthesizing polycyclic compounds: 1. Further modification of existing cyclic compounds to obtain the desired structure; 2. Utilizing highly unsaturated compounds such as 1,n-alkynes, 1,n-dienes, and 1,n-diynes to undergo cyclization reactions under certain conditions to construct polycyclic compounds. The former has higher specificity but requires prior design and synthesis of substrates. The latter is more efficient and can construct multiple chemical bonds simultaneously.

1,6-Diynes are a type of highly unsaturated compound containing two carbon-carbon triple bonds, which can synergistically act during reactions, allowing for various tandem cyclization reactions and exhibiting diverse reaction properties to yield different structural cyclic compounds. The synthesis of 1,6-diynes is simple, as structurally diverse 1,6-diynes can be synthesized through simple nucleophilic attacks, substitutions, or couplings of alkyne molecules. Under the influence of electrophilic reagents, transition metals, or bases, 1,6-diynes can undergo different pathways to obtain structurally diverse polycyclic compounds. In other words, the structure of 1,6-diynes themselves is diverse, and the products also exhibit diversity under different reaction conditions. Therefore, 1,6-diynes are a very useful class of organic synthesis intermediates.

### CONTENT OF THE INVENTION

According to a first aspect of the present application, a compound represented by a general formula (I) is provided. The 1,6-diyne is a type of compound with structural diversity and can be used as an important compound to synthesize compounds with great application value in fluorescent materials.

A compound represented by a general formula (I), and its tautomers, polymorphs, solvates, or salts thereof, 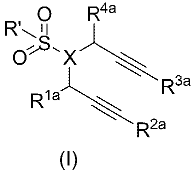
wherein, X is selected from nitrogen atom, phosphorus atom, arsenic atom, tellurium atom, and boron atom;
R' is selected from C₁-C₃₀ alkyl, substituted C₁-C₃₀ alkyl, C₆-C₃₀ aryl, substituted C₆-C₃₀ aryl, C₃-C₃₀ heteroaryl, substituted C₃-C₃₀ heteroaryl, amino, substituted amino and optionally inserted at any position by heteroatom groups selected from the following: CO, O, S, SO, SO₂, NR^{a}, -N=, =N-;
wherein, R^{1a}, R^{4a} are independently selected from hydrogen, deuterium, C₁-C₃₀ alkyl, substituted C₁-C₃₀ alkyl, C₆-C₃₀ aryl, substituted C₆-C₃₀ aryl, C₃-C₃₀ heteroaryl, substituted C₃-C₃₀ heteroaryl, phosphino, substituted phosphino, boryl, substituted boryl, silicon, substituted silicon, halogen, amino, substituted amino and optionally inserted at any position by heteroatom groups selected from the following: CO, O, S, SO, SO₂, NR^{a}, -N=, =N-;
R^{2a} is selected from C₆-C₃₀ aryl, substituted C₆-C₃₀ aryl, C₃-C₃₀ heteroaryl, substituted C₃-C₃₀ heteroaryl, C₁-C₁₀ alkenyl, C₁-C₁₀ substituted alkenyl;
R^{3a} is hydrogen, deuterium, C₁-C₃₀ alkyl, substituted C₁-C₃₀ alkyl, C₁-C₃₀ alkenyl, C₁-C₃₀ substituted alkenyl, C₁-C₃₀ alkynyl, substituted C₁-C₃₀ alkynyl, C₆-C₃₀ aryl, substituted C₆-C₃₀ aryl, C₃-C₃₀ heteroaryl, substituted C₃-C₃₀ heteroaryl" C₁-C₃₀ cycloalkyl, substituted C₁-C₃₀ cycloalkyl, C₁-C₃₀ heterocycloalkyl, substituted C₁-C₃₀ heterocycloalkyl, phosphino, halogen, silyl, boryl, germanium, arsenic, selenium and optionally inserted at any position by heteroatom groups selected from the following: CO, O, S, SO, SO₂, NR^{a}, -N=, =N-;
R^{a} is independently selected from H, alkyl or aryl.

Optionally, the substituents in the substituted C₆-C₃₀ aryl and the substituted C₃-C₃₀ heteroaryl are selected from alkyl, alkenyl, aldehyde, halogen, haloalkyl, ester-inserted alkyl, alkoxy, alkylthio, and substituted amino.

Optionally, the substituents in the substituted C₆-C₃₀ aryl and the substituted C₃-C₃₀ heteroaryl are selected from C₁-C₃₀ alkyl, C₁-C₃₀ alkenyl, C₁-C₃₀ aldehyde group, halogen, halogenated C₁-C₃₀ alkyl, ester-inserted C₁-C₃₀ alkyl, C₁-C₃₀ alkoxy, C₁-C₃₀ alkylthio, and phenyl-substituted amino.

Optionally, wherein R' is selected from C₆-C₃₀ aryl, C₆-C₁₀ alkyl-substituted C₆-C₃₀ aryl, C₆-C₃₀ aryl substituted with "halogen substituted C₆-C₁₀ alkyl", C₃-C₃₀ heteroaryl, C₆-C₁₀ alkyl substituted C₃-C₃₀ heteroaryl, C₃-C₃₀ heteroaryl substituted with "halogen substituted C₆-C₁₀ alkyl".

Optionally, R' is selected from C₆-C₃₀ aryl, C₃-C₃₀ heteroaryl

Optionally, R^{1a}, R^{4a} are independently selected from hydrogen, aryl, alkyl-substituted C₆-C₃₀ aryl, C₆-C₃₀ aryl substituted with "S atom-inserted alkyl", C₆-C₃₀ aryl substituted with "O atom-inserted alkyl", halogen-substituted C₆-C₃₀ aryl, aryl-substituted C₆-C₃₀ aryl, C₆-C₃₀ aryl substituted with "halogen-substituted alkyl", C₆-C₃₀ aryl substituted with "aryl-substituted amino", C₆-C₃₀ aryl substituted with "ester-inserted alkyl", and C₃-C₃₀ heteroaryl.

Optionally, R^{1a} is independently selected from phenyl, tert-butyl substituted phenyl, CH₃S-substituted phenyl, CH₃O-substituted phenyl, naphthyl, phenyl-substituted phenyl, phenyl substituted with at least one methyl, CF₃O-substituted phenyl, Br-substituted phenyl, phenyl substituted with "diphenyl-substituted amino", phenyl substituted with "ester -inserted methyl", dibenzothienyl.

Optionally, R^{2a} is selected from C₆-C₃₀ aryl, C₆-C₃₀ aryl substituted with "ester-inserted alkyl", alkyl-substituted C₆-C₃₀ aryl, C₆-C₃₀ aryl substituted with "oxygen atom-inserted alkyl", aryl-substituted C₆-C₃₀ aryl, C₃-C₃₀ heteroaryl, aldehyde-substituted C₆-C₃₀ aryl, halogen-substituted C₆-C₃₀ aryl, and alkenyl-substituted C₆-C₃₀ aryl.

Optionally, R^{2a} is selected from phenyl, naphthyl, phenyl substituted with "ester-inserted methyl", propyl-substituted phenyl, phenanthrenyl, phenyl substituted with "oxygen atom-inserted methyl", biphenyl, thienyl, formaldehyde-substituted phenyl, Cl-substituted phenyl, vinyl-substituted phenyl.

Optionally, R^{3a} is selected from hydrogen atom, deuterium atom, C₆-C₃₀ aryl, C₆-C₃₀ aryl substituted with "ester-inserted alkyl", C₆-C₃₀ aryl substituted with at least one alkyl.

Optionally, R^{3a} is selected from a hydrogen atom, a deuterium atom, phenyl, phenyl substituted with "ester-inserted methyl ", and phenyl substituted with at least one methyl.

Optionally, the tautomers have the general formula (II), the general formula (III) or the general formula (IV):

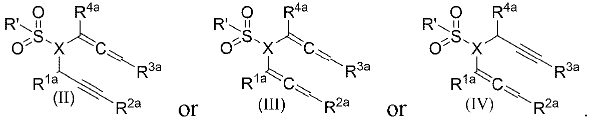

Optionally, the compound represented by formula (I) is selected from at least one of the following compounds:

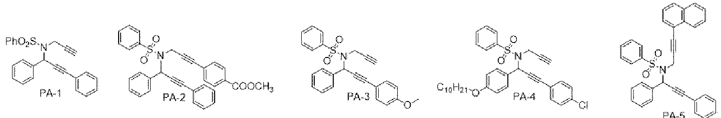

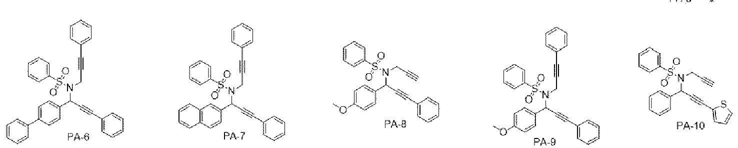

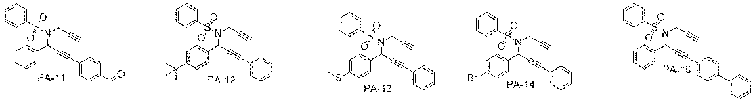

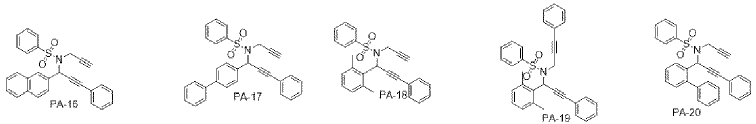

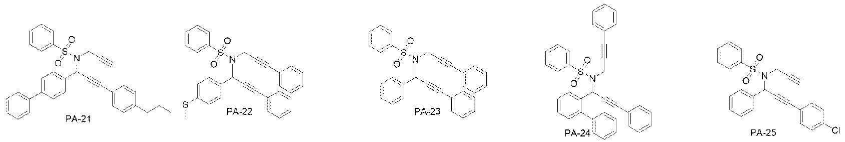

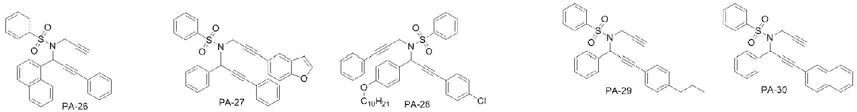

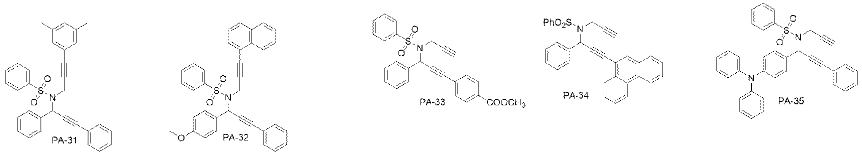

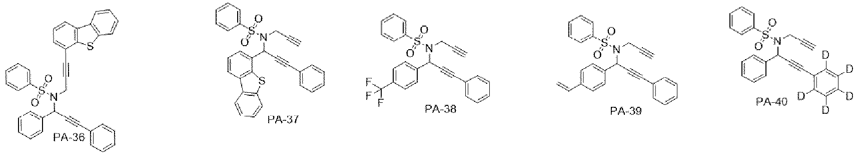

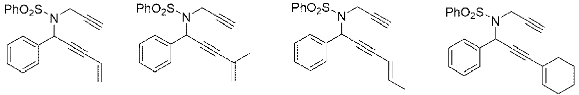

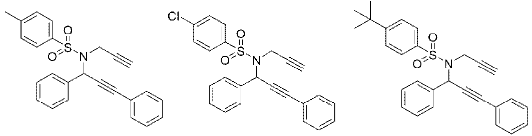

Optionally, the 1,6-diyne compound is used in preparation of mitochondrial fluorescence probes for mouse fibroblasts.

Optionally, the 1,6-diyne compound is used in preparation of mitochondrial fluorescence probes for mouse mononuclear macrophage leukemia cells or HER2-breast cancer overexpressing cells, or human liver cancer cell lines.

Optionally, the 1,6-diyne compound is used to detect the absence of enzymes in the respiratory chain.

The compound of the general formula (I) is used to detect substances containing active oxygen.

Optionally, the active oxygen includes at least one of sodium hypochlorite, hydrogen peroxide, or ozone.

Optionally, the compound represented by the general formula (I) is applied to a carrier such as a silica gel plate, a ceramic sheet, a polytetrafluoroethylene sheet, a sapphire substrate, a glass or a silicon dioxide substrate, and then a compound containing active oxygen (sodium hypochlorite, hydrogen peroxide, ozone, etc.) is dripped onto the carrier. The carrier is then heated for 30 seconds. The carrier will change from colorless to red, and under 365nm ultraviolet light, the fluorescence will gradually change from no fluorescence to strong fluorescence.

According to another aspect of the present application, a method for preparing the compound represented by the general formula (I) is provided.

The preparation method of the above-mentioned compound comprises the following steps:
reacting a raw material containing the compounds represented by the general formula (V) and the general formula (VI) to obtain the compound represented by the general formula (I);

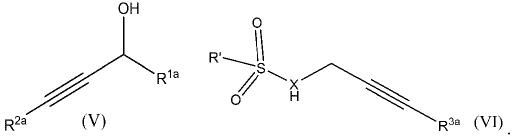

Optionally, a molar ratio of the compounds represented by the general formula (V) and the general formula (VI) is in a range from 1:1 to 1:5.

Optionally, the reaction is carried out in the presence of an acid reagent; the acid reagent includes p-toluenesulfonic acid, phenylsulfonic acid, p-nitrobenzenesulfonic acid, methanesulfonic acid, ferric chloride, or aluminum trichloride.

Optionally, the reaction temperature is in a range from 25 °C to 100°C; the reaction time is in a range from 0.1 h to 48 h.

Optionally, the preparation method of the compound represented by the general formula (V) includes the following steps:
reacting a raw material containing the compounds represented by the general formula (VII) and the general formula (VIII) to obtain the compound represented by the general formula (V);

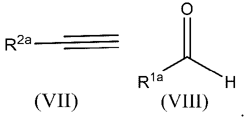

Optionally, a molar ratio of the compounds represented by the general formula (VII) and the general formula (VIII) is in a range from 1:1 to 1:5.

Optionally, the reaction is carried out in the presence of a nucleophilic substitution reagent; the nucleophilic substitution reagent includes n-butyllithium, sec-butyllithium, tert-butyllithium, methyllithium, diisopropylamino lithium, and bis(trimethylsilyl)amino lithium.

Optionally, the reaction temperature is in a range from -78 °C to 50 °C; the reaction time is in a range from 0.1h to 24h.

As an embodiment, the preparation method of the compound contains the following steps: 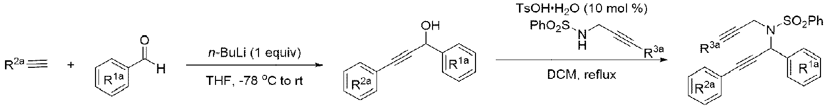 the main step is the nucleophilic substitution of alkynes with aldehyde compounds to obtain propargyl alcohol compounds. The propargyl alcohol compounds are then coupled with N-propargyl sulfonamide compounds to synthesize the desired compounds.

According to another aspect of the present application, a 3-arylbenzisoisoindole compound and its tautomers, polymorphs, solvates, or salts thereof are provided.

A 3-arylbenzisoisoindole compound and its tautomers, polymorphs, solvates, or salts thereof, wherein the 3-arylbenzisoisoindole compound has the structural formula described in formula (A): 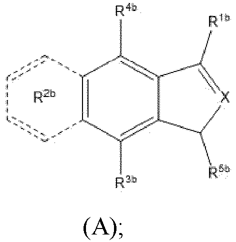
wherein X is one selected from nitrogen atom, phosphorus atom, arsenic atom, tellurium atom, and boron atom;
R^{1b} and R^{5b} have the same scope as R^{1a} in claim 1; R^{3b} and R^{4b} have the same scope as R^{1a} in claim 1; R^{2b} is selected from C₆-C₃₀ aryl, substituted C₆-C₃₀ aryl, C₃-C₃₀ heteroaryl, substituted C₃-C₃₀ heteroaryl.

According to another aspect of the present application, a preparation method of the 3-arylbenzisoisoindole compound represented by the general formula (IX) comprises the following steps:
reacting a mixture containing a 1,6-diyne compound, a base and an organic solvent I to obtain the 3-arylbenzisoindole compound;
the 1,6-diyne compound is selected from the compounds represented by the general formula (I).

Optionally, the base is at least one selected from cesium carbonate, potassium carbonate and sodium carbonate.

Optionally, the structural formula of the compound represented by the 3-arylbenzisoisoindole compound represented by the general formula (IX) is selected from the following compounds: 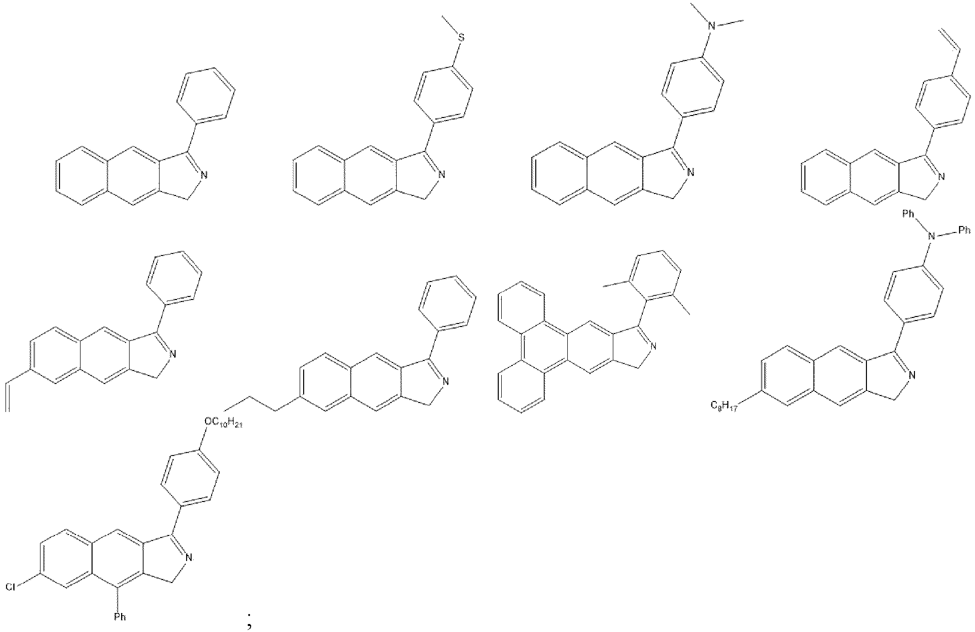 wherein, from left to right and from top to bottom, they are numbered: IM-1, IM-2, IM-3, IM-4, IM-5, IM-6, IM-7, IM-8, and IM-9.

Optionally, the organic solvent I is at least one selected from methanol, isopropyl alcohol, n-butanol, toluene and xylene.

Optionally, a molar ratio of the 1,6-diyne compound to the base is in a range from 1:0.1 to 1:10.

Optionally, a concentration of the 1,6-diyne compound is in a range from 20mM to 1M.

Optionally, the molar ratio of the 1,6-diyne compound to the base is in a range from 1:1 to 1:10.

Optionally, conditions for the reaction III are as follows: reaction temperature is in a range from 25°C to 150°C; reaction time is in a range from 0.5h to 72h.

Optionally, the reaction atmosphere is an inert gas.

According to another aspect of the present application, a benzisoindole dimer compound is provided.

A benzisoindole dimer compound, wherein the benzisoindole dimer compound has the structure shown in the following formula (I): 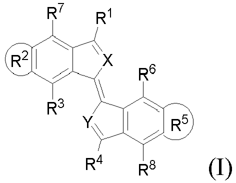
wherein, X and Y are independently selected from nitrogen atom, phosphorus atom, arsenic atom, tellurium atom, and boron atom;
R¹and R⁴ are the same or different; R¹and R⁴ have the same scope as R^{1a} in claim 1;
R² and R⁵ are the same or different; R² and R⁵ have the same scope as R^{2b} in claim 7;
R³ and R⁶ are the same or different; R⁷ and R⁸ are the same or different; R³, R⁶, R⁷ and R⁸ have the same scope as R^{1a} in claim 1.

Optionally, the benzisoindole dimer compound has a structure shown in general formula (II); 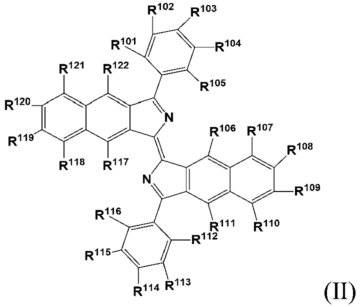
wherein R¹⁰¹, R¹⁰², R¹⁰³, R¹⁰⁴, R¹⁰⁵, R¹⁰⁶, R¹⁰⁷, R¹⁰⁸, R¹⁰⁹, R¹¹⁰, R¹¹¹, R¹¹², R¹¹³, R¹¹⁴, R¹¹⁵, R¹¹⁶, R¹¹⁷, R¹¹⁸, R¹¹⁹, R¹²⁰, R¹²¹, R¹²² are independently selected from hydrogen atom, C₁-C₁₅ alkyl, substituted C₁-C₁₅ alkyl, C₁-C₁₅ alkenyl, substituted C₁-C₁₅ alkenyl, C₁-C₁₅ alkynyl, substituted C₁-C₁₅ alkynyl, C₆-C₃₀ aryl, substituted C₆-C₃₀ aryl, C₃-C₃₀ heteroaryl, substituted C₃-C₃₀ heteroaryl, aldehyde, phosphine, halogen, silyl, boron, germanium, arsenic, selenium and optionally inserted at any position by a heteroatom group selected from the following: CO, O, S, SO, SO₂, N, NR^{a}, -N=, =N-; any two adjacent groups among R¹⁰¹, R¹⁰², R¹⁰³, R¹⁰⁴, R¹⁰⁵, R¹⁰⁶, R¹⁰⁷, R¹⁰⁸, R¹⁰⁹, R¹¹⁰, R¹¹¹, R¹¹², R¹¹³, R¹¹⁴, R¹¹⁵, R¹¹⁶, R¹¹⁷, R¹¹⁸, R¹¹⁹, R¹²⁰, R¹²¹, R¹²² can form a ring;
R^{a} is independently selected from H, alkyl or aryl.

Optionally, R¹⁰¹, R¹⁰², R¹⁰³, R¹⁰⁴, R¹⁰⁵, R¹⁰⁶, R¹⁰⁷, R¹⁰⁸, R¹⁰⁹, R¹¹⁰, R¹¹¹, R¹¹², R¹¹³, R¹¹⁴, R¹¹⁵, R¹¹⁶, R¹¹⁷, R¹¹⁸, R¹¹⁹, R¹²⁰, R¹²¹, R¹²² are independently selected from hydrogen atom, C₁-C₁₅ alkyl, halogen-substituted C₁-C₁₅ alkyl, aryl-substituted C₁-C₁₅ alkyl, S-inserted C₁-C₁₅ alkyl, O-inserted C₁-C₁₅ alkyl, ester-inserted C₁-C₁₅ alkyl, C₁-C₁₅ alkenyl, C₆-C₃₀ aryl, alkyl-substituted C₆-C₃₀ aryl, C₃-C₃₀ heteroaryl, substituted C₃-C₃₀ heteroaryl, and optionally inserted at any position by the following heteroatom groups: CO, O, S, SO, SO₂, N, NR^{a}, -N=, =N-; any two adjacent groups among R¹⁰¹, R¹⁰², R¹⁰³, R¹⁰⁴, R¹⁰⁵, R¹⁰⁶, R¹⁰⁷, R¹⁰⁸, R¹⁰⁹, R¹¹⁰, R¹¹¹, R¹¹², R¹¹³, R¹¹⁴, R¹¹⁵, R¹¹⁶, R¹¹⁷, R¹¹⁸, R¹¹⁹, R¹²⁰, R¹²¹, R¹²² can form a ring;
R^{a} is independently selected from H, alkyl or aryl.

Optionally, the benzisoindole dimer compound has a structure shown in general formula (III); 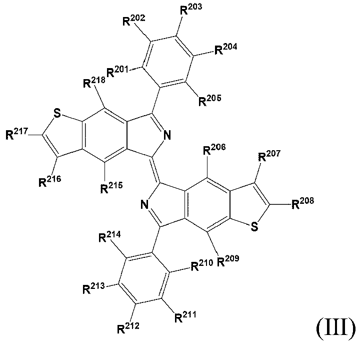
wherein R²⁰¹, R²⁰², R²⁰³, R²⁰⁴, R²⁰⁵, R²⁰⁶, R²⁰⁷, R²⁰⁸, R²⁰⁹, R²¹⁰, R²¹¹, R²¹², R²¹³, R²¹⁴, R²¹⁵, R²¹⁶, R²¹⁷, and R²¹⁸ are independently selected from hydrogen atom, C₁-C₁₅ alkyl, substituted C₁-C₁₅ alkyl, C₁-C₁₅ alkenyl, substituted C₁-C₁₅ alkenyl, C₁-C₁₅ alkynyl, substituted C₁-C₁₅ alkynyl, C₆-C₃₀ aryl, substituted C₆-C₃₀ aryl, C₃-C₃₀ heteroaryl, substituted C₃-C₃₀ heteroaryl, aldehyde, phosphine, halogen, silyl, boron, germanium, arsenic, selenium and optionally inserted at any position by a heteroatom group selected from the following: CO, O, S, SO, SO₂, N, NR^{a}, -N=, =N-; any two adjacent groups among R²⁰¹, R²⁰², R²⁰³, R²⁰⁴, R²⁰⁵, R²⁰⁶, R²⁰⁷, R²⁰⁸, R²⁰⁹, R²¹⁰, R²¹¹, R²¹², R²¹³, R²¹⁴, R²¹⁵, R²¹⁶, R²¹⁷, R²¹⁸ can form a ring;
R^{a} is independently selected from H, alkyl or aryl.

Optionally, R²⁰¹, R²⁰², R²⁰³, R²⁰⁴, R²⁰⁵, R²⁰⁶, R²⁰⁷, R²⁰⁸, R²⁰⁹, R²¹⁰, R²¹¹, R²¹², R²¹³, R²¹⁴, R²¹⁵, R²¹⁶, R²¹⁷, and R²¹⁸ are independently selected from hydrogen atom, C₁-C₁₅ alkyl, halogen-substituted C₁-C₁₅ alkyl, aryl-substituted C₁-C₁₅ alkyl, S-inserted C₁-C₁₅ alkyl, O-inserted C₁-C₁₅ alkyl, ester-inserted C₁-C₁₅ alkyl, C₁-C₁₅ alkenyl, C₆-C₃₀ aryl, alkyl-substituted C₆-C₃₀ aryl, C₃-C₃₀ heteroaryl, substituted C₃-C₃₀ heteroaryl, and optionally inserted at any position by the following heteroatom groups: CO, O, S, SO, SO₂, N, NR^{a}, -N=, =N-; any two adjacent groups among R²⁰¹, R²⁰², R²⁰³, R²⁰⁴, R²⁰⁵, R²⁰⁶, R²⁰⁷, R²⁰⁸, R²⁰⁹, R²¹⁰, R²¹¹, R²¹², R²¹³, R²¹⁴, R²¹⁵, R²¹⁶, R²¹⁷, and R²¹⁸ can form a ring;
R^{a} is independently selected from H, alkyl or aryl.

According to another aspect of the present application, a method for preparing a benzisoindole dimer compound contains the following steps:
reacting a mixture containing a 3-aryl benzoisoindole compound, an oxidant, and an organic solvent II to obtain the benzoisoindole dimer compound;
the 3-aryl benzoisoindole compound is selected from the 3-aryl benzoisoindole compound according to claim 28 and its tautomeric isomers, polymorphs, solvates, or salts thereof.

Optionally, the oxidant is at least one selected from 2,2,6,6-tetramethylpiperidine nitrogen oxide, oxygen, sodium hypochlorite, aqueous hydrogen peroxide solution, tert-butanol peroxide, lauroyl peroxide, benzoyl peroxide, tert-butyl perbenzoate, m-chloroperoxybenzoic acid, peracetic acid, and di-tert-butyl peroxide.

Optionally, the organic solvent II is at least one selected from methanol, ethanol, isopropanol, p-xylene, n-butanol, ethyl acetate, acetonitrile, N,N-dimethylformamide and dimethyl sulfoxide.

Optionally, conditions for reaction II are as follows: reaction temperature is in a range from 25 °C to 150°C; reaction time is in a range from 0.5h to 72h.

According to another aspect of the present application, use of the compound represented by the general formula (I) and/or the compound obtained by the preparation method in the preparation of mitochondrial fluorescent probes, in the detection of substances containing active oxygen and in cell imaging are provided.

Optionally, the method for preparing the mitochondrial fluorescent probe contains the following steps:
incubating the culture dish containing the 1,6-diyne compound and mouse fibroblasts.

Optionally, volume of a solution of the 1,6-diyne compound is in a range from 1 µL to 1000 µL.

Optionally, concentration of a solution of the 1,6-diyne compound is in a range from 1 µM to 1000 µM.

Optionally, conditions for incubation are as follows:
reaction temperature is in a range from 5 °C to 50°C; reaction time is in a range from 0.1h to 48h.

Optionally, the compound represented by the general formula (I) is used in preparation of mitochondrial fluorescent probes of mouse fibroblasts.

Optionally, the compound represented by the general formula (I) is used in preparation of mitochondrial fluorescent probes for mouse mononuclear macrophage leukemia cells or HER2-breast cancer overexpression cells or human liver cancer cell lines.

Optionally, the active oxygen includes at least one of sodium hypochlorite, hydrogen peroxide, and ozone.

According to another aspect of the present application, use of the 3-arylbenzoisoindole compound obtained by the preparation method and/or the 3-arylbenzoisoindole compound and its tautomers, polymorphs, solvates, or their salts in lysosomal fluorescent probe, autophagy lysosome fluorescent probe, synthesis of benzisoindole dimer compound, optical super-resolution microscopy, confocal microscopy, wide-field microscopy, fluorescence lifetime imaging microscopy, fluorescence resonance energy transfer microscopy, super-resolution optical wave imaging, fluorescence activation localization microscopy, and light-emitting device are provided.

According to another aspect of the present application, use of the benzisoindole dimer compound obtained by the preparation method according to any one of claims 29 to 32 and/or the benzisoindole dimer compound according to any one of claims 33 to 37 in mitochondrial fluorescence probes, optical super-resolution microscopy, confocal microscopy, wide-field microscopy, fluorescence lifetime imaging microscopy, fluorescence resonance energy transfer microscopy, super-resolution optical fluctuation imaging, fluorescence photoactivated localization microscopy, light-emitting devices, cellular imaging, and fluorescent dyes are provided.

Optionally, the benzisoindole dimer compound is produced by the compound represented by the general formula (I) according to any one of claims 1 to 12 during cell incubation.

Optionally, excitation wavelength of the benzisoindole dimer compound is in a range from 220 nm to 1000 nm.

Optionally, emission wavelength of the benzisoindole dimer compound is in a range from 400 nm to 800 nm.

In the present application, "alkyl" refers to a group obtained by losing a H atom from an alkane.

"Alkenyl" refers to a group obtained by losing a H atom from an alkene.

"Alkynyl" refers to a group obtained by losing a H atom from an alkyne.

"Cycloalkyl" refers to a group obtained by losing a H atom from a cycloalkane.

"Heterocycloalkyl" refers to a group obtained by losing a H atom from a heterocycloalkane.

"Aryl" refers to a group obtained by losing a H atom from an aromatic hydrocarbon.

"Heteroaryl" refers to a group obtained by losing a H atom from a heteroaromatic hydrocarbon.

"Substituted alkyl," "substituted aryl," and the like refer to alkyl substituted with any group, and aryl substituted with any group, respectively.

"inserted at any position by heteroatom groups" means that heteroatom groups can be inserted into the group by replacing a hydrogen atom or a carbon atom, for example, "O-inserted methyl is CH₃O-", "S-inserted methyl is CH₃S-", " NH inserted methyl group is CH₃NH-" and "N-inserted phenyl is a pyridyl", etc.

"Alkyl-substituted aryl", "aryl-substituted aryl" and the like respectively represent aryl containing alkyl substituent(s) and aryl containing aryl substituent(s), for example 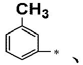 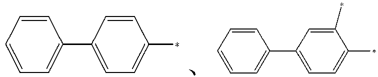 etc.; wherein "*" represents the connection point of the group.

In the present application, unless otherwise specified, "alkyl", "aryl" and the like may have any number of connection points; generally, there may be 1, 2, 3, 4, or 5 connection points.

" C₁-C₃₀" refers to the number of carbon atoms.

"Halogen-substituted C₁-C₃₀ alkyl" refers to an alkyl group having 1 to 30 carbon atoms and substituted with halogen carbon atoms is between 1 and 30.

"Halogen" refers to fluorine, chlorine, bromine, and iodine.

"optionally inserted at any position by a heteroatom group selected from the following: CO, O, S, SO, SO₂, N, NR^{a}, -N=, =N-" means that the heteroatom group can be inserted at any position under the premise of satisfying the valence bond rules, for example, between any C-C, C-S, C-O, or O-O bonds.

"Salt" refers to acidic and/or basic salts formed with inorganic and/or organic acids and bases. Furthermore, when the compound in the present application contains a basic moiety (such as, but not limited to, pyridine or imidazole) and an acidic moiety (such as, but not limited to, carboxylic acid), zwitterions ("inner salts") may form and are included in the term "salt" as used herein.

"Solvate" includes, for example, hydrates.

The structural formulas described in the present application are intended to include all isomeric forms (such as enantiomers, diastereomers, and geometric isomers (or conformational isomers)): for example, R-S configurations with asymmetric centers, and (Z), (E) isomers of double bonds, etc. Therefore, single stereochemical isomers or mixtures of enantiomers, diastereomers or geometric isomers (or conformational isomers) of the compounds in the present application are within the scope of the present application.

"Tautomers" refer to structural isomers with different energies that can interconvert by overcoming low energy barriers. For example, proton tautomers (i.e., prototropic) include interconversions by migration of a proton. Valence tautomers include interconversions by reorganization of some of the bonding electrons.

"Solvate" refers to a complex formed by the coordination of the compound in the present application with solvent molecule(s) in a specific ratio.

The beneficial effects that can be achieved by this application include:
1) The 1,6-diyne compounds provided in the present application have variable structures, are easy to be synthesized, and are suitable for industrial production; they can be used for the synthesis of a class of benzoisoindole dimer compounds and their derivatives, cell imaging, and detecting reactive oxygen species (such as sodium hypochlorite, hydrogen peroxide, ozone, etc.).
2) The 3-arylbenzoisoindole compound provided in the present application can be used as an important compound and play an important role in pharmaceuticals or fluorescent materials.
3) The benzoisoindole dimer compounds provided in the present application have different excitation and emission wavelengths, which can meet the needs of various mitochondrial fluorescent probes; they can effectively localize mitochondria as probe molecules; and they exhibit good adaptability in most microenvironments as probe molecules.

### DESCRIPTION OF THE DRAWINGS

FIG. 1 shows a color development of the compound PA-1 under fluorescence.
FIG. 2 is a mass spectrometry graph of the benzoindole dimer.
FIG. 3 is a spectrometry graph of the cell lysate extract.
FIG. 4 is a fluorescence emission spectrum of the compound FL-1 in relation to its concentration and luminescence intensity.
FIG. 5 is a fluorescence emission spectrum of the compound FL-1 in relation to its concentration and luminescence intensity.
FIG. 6 is a fluorescence emission spectrum of the compound FL-1 at a concentration of 0.05 micrograms per milliliter.
FIG. 7 is a cyclic voltammetry curve of the compound FL-1.
FIG. 8 shows the cell imaging after treatment with cytc-290 in Example 22; wherein b1 is a red channel MP-1 staining of the cells; b2 is a bright field; b3 is a blue channel Hoechst staining of the cells; b4 is a green channel MTG staining of the cells; b5 is a merged image of b1, b2, and b3.
FIG. 9 shows the mitochondrial imaging of L292 cells using the MP-1 probe molecule in Example 19, wherein a3 is a bright field cell photo; a4 is a red channel MP-1 cell staining; a5 is a red channel two-photon cell photo.
FIG. 10 shows the mitochondrial imaging of L292 cells using the MP-2 probe molecule in Example 20, wherein b4 is a bright field cell photograph; b5 is a green channel MP-2 cell staining image; and b6 is a two-photon green channel MP-2 cell staining image.
FIG. 11 shows the mitochondrial imaging of SKBR3 cells using the MP-1 probe molecule in Example 21, wherein a is a bright field cell photo; b is a red channel MP-1 cell staining image.
FIG. 12 shows the mitochondrial imaging of Raw264.7 cells using the MP-1 probe molecule in Example 21, wherein a is a bright field cell photo; b is a red channel MP-1 cell staining image; c is the green channel MTG cell staining image.
FIG. 13 shows the mitochondrial imaging of HuH7-1 cells using the MP-1 probe molecule in Example 21, wherein a is a bright field cell photo; b is a red channel MP-1 cell staining; c is a green channel MTG cell staining.

### SPECIFIC IMPLEMENTATIONS

The present application is described in detail below with reference to the Examples, but the present application is not limited to these Examples.

Unless otherwise specified, the raw materials and catalysts in the Examples of the present application are purchased through commercial channels.

Among them, IPA refers to isopropanol; nBuOH refers to n-butanol; p-xylene refers to para-xylene.

The analytical methods in the Examples of the present application are as follows:
Nuclear magnetic resonance analysis is performed using Bruker-BioSpin AVANCE III HD and JEOL ECZ600S instruments.

Mass spectrometry analysis is performed using a Thermo Fisher Scientific LTQ FTICR-MS instrument.

Cell fluorescence labeling analysis is performed using a Zeiss LSM 880 confocal fluorescence microscope.

Ultraviolet-visible absorption and emission spectrum analysis is performed using a Shimadzu UV-1900 and an Edinburgh FS5-NIR.

Cyclic voltammetry analysis is performed using a Shanghai Chenhua electrochemical analyzer.

Hank's buffer solution consists of 1.26 mM CaCl₂, 0.49 mM MgCl₂, 0.41 mM MgSO₄, 5.33 mM KCl, 0.44 mM KH₂PO₄, 4.17 mM NaHCO₃, 138 mM NaCl, 0.34 mM Na₂HPO₄, 5.55 mM glucose, pH = 7.4.

Silent siRNA (cytc-290, cytc-436) is purchased from Suzhou GenePharma Co., Ltd.

The calculation formulas for the yields of compound IM-(1~9) and dimeric compound FL-(1-9) in the Examples of the present application are as follows: Yield of compound IM-(1-9) = (molar mass of product/molar mass of raw material) * 100%. Yield of dimeric compound FL-(1-9) = (molar mass of product/molar mass of raw material) * 2 * 100%.

### Example 1 Preparation of 1,6-diyne compounds

The compounds in Table 1 are prepared by the following method:

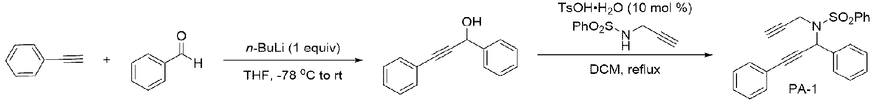

In a dry 50 mL round-bottom flask equipped with a stirrer, phenylacetylene (5 mmol, 1 equiv.) and 10 mL of tetrahydrofuran are added. The round-bottom flask is cooled to -78 °C under an inert gas atmosphere, and then n-butyllithium (2.5 M in THF, 2 mL, 5 mmol, 1 equiv.) is slowly added dropwise. The reaction is allowed to return to room temperature and stirred for 30 minutes, then cooled again to -78 °C. Benzaldehyde (5 mmol, 1 equiv.) is dissolved in tetrahydrofuran and slowly added to the reaction mixture. After the addition is complete, the reaction is returned to room temperature and stirred for 30 minutes. After the reaction is complete, the reaction is quenched with saturated ammonium chloride aqueous solution, and the aqueous phase is extracted three times with ethyl acetate. The organic phases are combined, dried over magnesium sulfate, filtered, and spin-dried to obtain a crude product of propargyl alcohol.

The crude product of propargyl alcohol and N-propargyl benzenesulfonamide (6 mmol, 1.2 equiv.) are dissolved in 10 mL of dichloromethane under an inert gas atmosphere. TsOH·H₂O (0.5 mmol, 10 mol%) is then added, and the reaction is heated to reflux overnight. After the reaction is complete, the reaction is quenched with a saturated aqueous solution of NaHCO₃, and the aqueous phase is extracted three times with ethyl acetate. The organic phases are combined, dried over magnesium sulfate, filtered, and spin-dried to obtain the crude product of propargyl alcohol. The crude product is purified by silica gel column chromatography to yield MP-1 (yield 57%).

The preparation method of PA-2 compound is similar to that of PA-1, except that N-propargylbenzenesulfonamide is replaced with the following structure

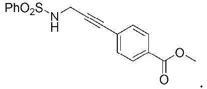

The preparation method of the PA-3 compound is similar to that of the PA-1 compound, except that phenylacetylene is replaced by 4-methoxyphenylacetylene.

The preparation method of the PA-4 compound is similar to that of the PA-1, except that phenylacetylene is replaced by 4-chlorophenylacetylene, and benzaldehyde is replaced by 4-decyloxybenzaldehyde.

The preparation method of PA-5 compound is similar to that of PA-1, except that N-propargylbenzenesulfonamide is replaced with the following structure

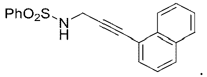

The preparation method of PA-6 compound is similar to that of PA-1, except that benzaldehyde is replaced by 4-phenylbenzaldehyde and N-propargylbenzenesulfonamide is replaced by the following structure

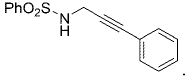

The preparation method of PA-7 compound is similar to that of PA-1, except that benzaldehyde is replaced by 2-naphthaldehyde and N-propargylbenzenesulfonamide is replaced by the following structure

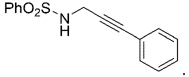

The preparation method of PA-8 compound is similar to that of PA-1, except that benzaldehyde is replaced by 4-methoxyformaldehyde.

The preparation method of PA-9 compound is similar to that of PA-1, except that benzaldehyde is replaced by 4-methoxyformaldehyde and N-propargylbenzenesulfonamide is replaced by the following structure

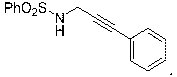

The preparation method of the PA-10 compound is similar to that of the PA-1 compound, except that benzaldehyde is replaced by thiophene-2-carboxaldehyde.

The preparation method of the PA-11 compound is similar to that of the PA-1 compound, except that phenylacetylene is replaced by 4-formylphenylacetylene.

The preparation method of the PA-12 compound is similar to that of the PA-1 compound, except that benzaldehyde is replaced by 4-tert-butylbenzaldehyde.

The preparation method of the PA-13 compound is similar to that of the PA-1 compound, except that benzaldehyde is replaced by 4-methylthiobenzaldehyde.

The preparation method of the PA-14 compound is similar to that of the PA-1 compound, except that benzaldehyde is replaced by 4-bromobenzaldehyde.

The preparation method of the PA-15 compound is similar to that of the PA-1 compound, except that phenylacetylene is replaced by 4-phenylphenylacetylene.

The preparation method of the PA-16 compound is similar to that of the PA-1 compound, except that phenylacetylene is replaced by 2-naphthylacetylene.

The preparation method of PA-17 compound is similar to that of PA-1, except that benzaldehyde is replaced by 4-phenylbenzaldehyde.

The preparation method of the PA-18 compound is similar to that of the PA-1 compound, except that benzaldehyde is replaced by 2,6-dimethylbenzaldehyde

The preparation method of PA-19 compound is similar to that of PA-1, except that benzaldehyde is replaced by 2,6-dimethylbenzaldehyde and N-propargylbenzenesulfonamide is replaced by the following structure

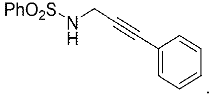

The preparation method of the PA-20 compound is similar to that of the PA-1 compound, except that benzaldehyde is replaced by 2-phenylbenzaldehyde.

The preparation method of the PA-21 compound is similar to that of the PA-1 compound, except that benzaldehyde is replaced by 4-phenylbenzaldehyde, and phenylacetylene is replaced by 4-propylphenylacetylene.

The preparation method of PA-22 compound is similar to that of PA-1, except that benzaldehyde is replaced by 4-methylthiobenzaldehyde and N-propargylbenzenesulfonamide is replaced by the following structure

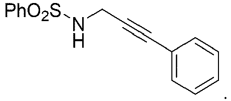

The preparation method of PA-23 compound is similar to that of PA-1, except that N-propargylbenzenesulfonamide is replaced with the following structure

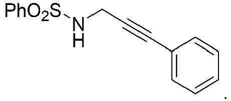

The preparation method of PA-24 compound is similar to that of PA-1, except that benzaldehyde is replaced by 2-phenylbenzaldehyde and N-propargylbenzenesulfonamide is replaced by the following structure

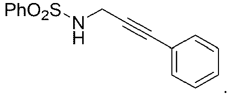

The preparation method of the PA-25 compound is similar to that of the PA-1 compound, except that phenylacetylene is replaced by 4-chlorophenylacetylene.

The preparation method of PA-26 compound is similar to that of PA-1, except that benzaldehyde is replaced by 1-naphthaldehyde.

The preparation method of PA-27 compound is similar to that of PA-1, except that N-propargylbenzenesulfonamide is replaced with the following structure

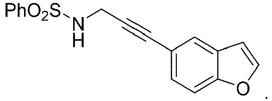

The preparation method of PA-28 compound is similar to that of PA-1, except that benzaldehyde is replaced by 4-decyloxyformaldehyde, phenylacetylene is replaced by 4-chlorophenylacetylene, and N-propargylbenzenesulfonamide is replaced by the following structure

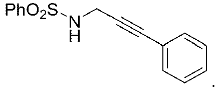

The preparation method of the PA-29 compound is similar to that of the PA-1 compound, except that phenylacetylene is replaced by 4-propylphenylacetylene.

The preparation method of the PA-30 compound is similar to that of the PA-1 compound, except that phenylacetylene is replaced by 2-naphthyleneacetylene.

The preparation method of PA-31 compound is similar to that of PA-1, except that N-propargylbenzenesulfonamide is replaced with the following structure

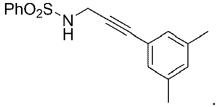

The preparation method of PA-32 compound is similar to that of PA-1, except that benzaldehyde is replaced by 4-methoxybenzaldehyde and N-propargylbenzenesulfonamide is replaced by the following structure

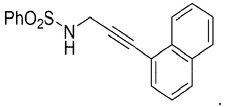

The preparation method of the PA-33 compound is similar to that of the PA-1 compound, except that phenylacetylene is replaced by 4-methoxycarbonylphenylacetylene.

The preparation method of the PA-34 compound is similar to that of the PA-1 compound, except that phenylacetylene is replaced by 9-ethynylphenanthrene.

The preparation method of the PA-35 compound is similar to that of the PA-1 compound, except that benzaldehyde is replaced by 4-diphenylaminobenzaldehyde.

The preparation method of PA-36 compound is similar to that of PA-1, except that N-propargylbenzenesulfonamide is replaced with the following structure

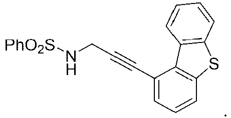

The preparation method of the PA-37 compound is similar to that of the PA-1 compound, except that benzaldehyde is replaced by 4-formyldibenzothiophene.

The preparation method of PA-38 compound is similar to that of PA-1, except that benzaldehyde is replaced by 4-trifluoromethylbenzaldehyde.

The preparation method of the PA-39 compound is similar to that of the PA-1 compound, except that benzaldehyde is replaced by 4-vinylbenzaldehyde.

The preparation method of the PA-40 compound is similar to that of PA-1, except that phenylacetylene is replaced with 2,3,4,5,6-pentadeuterated phenylacetylene.

The results of the NMR test are shown in the following table:

| NO. | The compounds | Nuclear magnetic resonance data |
|---|---|---|
| PA-1 | 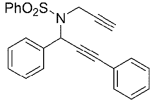 | ¹H NMR (600 MHz, Chloroform-*d*) δ 8.09 - 8.02 (m, 2H), 7.72 - 7.66 (m, 2H), 7.58 - 7.54 (m, 1H), 7.51 - 7.48 (m, 2H), 7.41 - 7.37 (m, 2H), 7.37 - 7.33 (m, 1H), 7.33 - 7.29 (m, 1H), 7.28 - 7.25 (m, 2H), 7.20 - 7.17 (m, 2H), 6.32 (s, 1H), 4.13 (dd, *J* = 18.4, 2.6 Hz, 1H), 3.75 (dd, *J =* 18.4, 2.5 Hz, 1H), 1.89 (t, *J =* 2.5 Hz, 1H). |
| PA-2 | 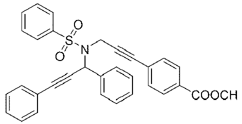 | ¹H NMR (600 MHz, CDC1₃) δ 8.09 (d, *J* = 7.2 Hz, 2H), 7.80 - 7.72 (m, 4H), 7.52 (t, *J* = 7.4 Hz, 1H), 7.48 - 7.38 (m, 4H), 7.35 (t, *J =* 7.4 Hz, 1H), 7.30 - 7.22 (m, 1H), 7.18 - 7.10 (m, 4H), 6.92 (d, *J =* 8.5 Hz, 2H), 6.39 (s, 1H), 4.43 (d, *J =* 18.5 Hz, 1H), 3.99 (d, *J =* 18.5 Hz, 1H), 3.88 (s, 3H). ¹³C NMR (151 MHz, CDCl₃) δ 166.59, 139.60, 135.77, 132.93, 131.77, 131.56, 129.39, 129.11, 128.87, 128.83, 128.78, 128.36, 128.28, 128.27, 127.26, 121.96, 89.04, 87.03, 84.25, 83.20, 53.73, 52.34, 34.64. |
| PA-3 | 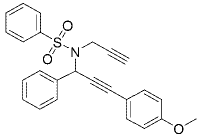 | ¹H NMR (600 MHz, CDCl₃) δ 8.06 - 8.03 (m, 2H), 7.72 - 7.65 (m, 2H), 7.58 - 7.54 (m, 1H), 7.51 - 7.47 (m, 2H), 7.40 - 7.33 (m, 3H), 7.12 (d, *J =* 8.9 Hz, 2H), 6.78 (d, *J =* 8.9 Hz, 2H), 6.29 (s, 1H), 4.11 (dd, *J* = 18.4, 2.6 Hz, 1H), 3.80 (s, 3H), 3.75 (dd, *J* = 18.4, 2.5 Hz, 1H), 1.88 (t, *J* = 2.5 Hz, 1H). ¹³C NMR (151 MHz, CDCl₃) δ 139.36, 135.88, 133.06, 132.95, 132.91, 128.79, 128.67, 128.63, 128.38, 128.30, 114.27, 113.91, 88.79, 81.80, 78.30, 72.66, 55.39, 53.94, 33.83. |
| PA-4 | 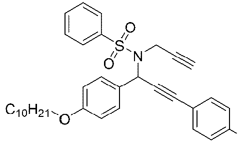 | ¹H NMR (600 MHz, CDCl₃) δ 8.06 - 8.00 (m, 2H), 7.57 - 7.51 (m, 3H), 7.50 - 7.44 (m, 2H), 7.23 (d, *J =* 8.7 Hz, 2H), 7.08 (d, *J* = 8.6 Hz, 2H), 6.89 (d, *J* = 8.8 Hz, 2H), 6.24 (s, 1H), 4.13 (dd, *J* = 18.5, 2.5 Hz, 1H), 3.95 (t, *J* = 6.6 Hz, 2H), 3.71 (dd, *J* = 18.5, 2.5 Hz, 1H), 1.90 (t, *J =* 2.5 Hz, 1H), 1.80 - 1.75 (m, 2H), 1.46 - 1.41 (m, 2H), 1.29 - 1.23 (m, 13H), 0.88 (t, *J=* 6.9 Hz, 3H). ¹³C NMR (151 MHz, CDCl₃) δ 159.62, 139.44, 134.75, 132.80, 132.71, 129.62, 128.75, 128.70, 128.31, 127.00, 120.74, 114.56, 87.30, 84.82, 78.33, 68.21, 53.17, 33.52, 31.99, 29.67, 29.66, 29.49, 29.42, 29.38, 29.31, 26.16, 22.78, 14.23. |
| PA-5 | 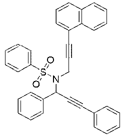 | ¹H NMR (600 MHz, CDCl₃) δ 8.15 - 8.10 (m, 2H), 7.88 (d, *J* = 8.3 Hz, 1H), 7.80 - 7.77 (m, 2H), 7.75 (d, *J* = 7.7 Hz, 1H), 7.71 (d, *J* = 8.2 Hz, 1H), 7.47 - 7.44 (m, 1H), 7.43 - 7.37 (m, 5H), 7.36 - 7.27 (m, 3H), 7.22 - 7.18 (m, 2H), 7.12 - 7.06 (m, 5H), 6.43 (s, 1H), 4.58 (d, *J* = 18.4 Hz, 1H), 4.15 (d, *J* = 18.5 Hz, 1H). ¹³C NMR (151 MHz, CDCl₃) δ 139.68, 135.90, 133.22, 132.98, 132.84, 131.81, 130.84, 128.89, 128.80, 128.78, 128.67, 128.66, 128.43, 128.25, 128.12, 128.08, 126.62, 126.37, 126.33, 124.94, 122.02, 120.30, 88.90, 88.71, 83.47, 83.14, 53.81, 35.02. |
| PA-6 | 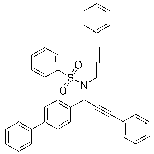 | ¹H NMR (600 MHz, CDCl₃) δ 8.12 - 8.07 (m, 2H), 7.83 - 7.78 (m, 2H), 7.61 (d, *J* = 8.4 Hz, 2H), 7.57 - 7.55 (m, 2H), 7.52 (d, *J* = 7.5 Hz, 1H), 7.48 - 7.43 (m, 4H), 7.36 (t, *J* = 7.4 Hz, 1H), 7.28 - 7.25 (m, 1H), 7.20 - 7.14 (m, 5H), 7.06 (t, *J* = 7.9 Hz, 2H), 6.90 (d, *J* = 9.2 Hz, 2H), 6.42 (s, 1H), 4.42 (d, *J* = 18.4 Hz, 1H), 4.08 (d, *J* = 18.4 Hz, 1H). ¹³ C NMR (151 MHz, CDCl₃) δ 141.65, 140.59, 139.62, 134.94, 132.83, 131.82, 131.66, 128.90, 128.86, 128.71, 128.25, 128.21, 128.10, 127.96, 127.61, 127.42, 127.27, 122.56, 122.06, 88.96, 85.03, 83.83, 83.24, 53.54, 34.82. |
| PA-7 | 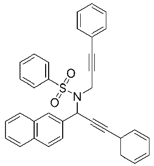 | ¹H NMR (600 MHz, CDCl₃) δ 8.24 (s, 1H), 8.17 - 8.10 (m, 2H), 7.90 - 7.81 (m, 4H), 7.55 - 7.50 (m, 3H), 7.47 (t, *J =* 8.0 Hz, 2H), 7.29 (dt, *J* = 8.8, 4.3 Hz, 1H), 7.23 - 7.19 (m, 4H), 7.19 - 7.14 (m, 1H), 7.04 (t, *J* = 7.9 Hz, 2H), 6.80 - 6.75 (m, 2H), 6.55 (s, 1H), 4.44 (d, *J* = 18.4 Hz, 1H), 4.03 (d, *J* = 18.4 Hz, 1H). ¹³C NMR (151 MHz, CDCl₃) δ 139.66, 133.49, 133.29, 133.13, 132.90, 131.94, 131.82, 131.64, 131.60, 128.89, 128.73, 128.44, 128.39, 128.29, 128.20, 128.13, 127.99, 127.81, 126.73, 126.51, 125.98, 122.49, 122.12, 89.16, 85.02, 83.83, 83.29, 53.92, 34.74. |
| PA-8 | 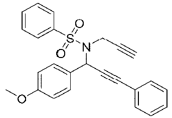 | ¹H NMR (600 MHz, CDCl₃) δ 8.10 - 8.06 (m, 2H), 7.73 - 7.69 (m, 2H), 7.60 - 7.55 (m, 3H), 7.54 - 7.48 (m, 4H), 7.47 - 7.43 (m, 2H), 7.43 - 7.39 (m, 2H), 7.39 - 7.34 (m, 2H), 7.27 (d, *J* = 8.5 Hz, 2H), 6.35 (s, 1H), 4.16 (dd, *J =* 18.4, 2.6 Hz, 1H), 3.78 (dd, *J* = 18.4, 2.5 Hz, 1H), 1.92 (t, *J* = 2.5 Hz, 1H). ¹³C NMR (151 MHz, CDCl₃) δ 141.53, 140.22, 139.36, 135.67, 133.02, 132.09, 132.00, 129.04, 128.87, 127.11, 127.00, 121.04, 88.70, 83.93, 78.24, 72.80, 53.85, 33.87. |
| PA-9 | 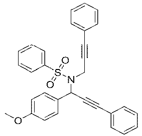 | ¹H NMR (600 MHz, Chloroform-*d*) δ 8.11 - 8.06 (m, 2H), 7.66 - 7.61 (m, 2H), 7.52 - 7.48 (m, 1H), 7.44 (t, *J* = 7.6 Hz, 2H), 7.26 - 7.23 (m, 1H), 7.19 (t, *J =* 7.5 Hz, 1H), 7.15 (t, *J =* 7.8 Hz, 2H), 7.12 - 7.08 (m, 4H), 6.95 - 6.89 (m, 4H), 6.32 (s, 1H), 4.39 (d, *J* = 18.4 Hz, 1H), 3.97 (d, *J* = 18.4 Hz, 1H), 3.79 (s, 3H). |
| PA-10 | 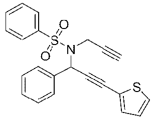 | ¹H NMR (600 MHz, Chloroform-*d*) δ 8.05 - 8.01 (m, 2H), 7.67 - 7.64 (m, 2H), 7.59 - 7.55 (m, 1H), 7.52 - 7.48 (m, 2H), 7.41 - 7.38 (m, 2H), 7.37 - 7.34 (m, 1H), 7.23 (dd, *J* = 5.1, 1.2 Hz, 1H), 7.02 (dd, *J* = 3.7, 1.0 Hz, 1H), 6.93 (dd, *J* = 5.2, 3.6 Hz, 1H), 6.32 (s, 1H), 4.13 (dd, *J* = 18.5, 2.6 Hz, 1H), 3.71 (dd, *J* = 18.4, 2.5 Hz, 1H), 1.91 (t, *J* = 2.5 Hz, 1H). |
| PA-11 | 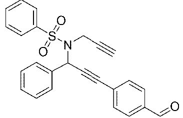 | ¹H NMR (600 MHz, Chloroform-*d*) δ 9.97 (s, 1H), 8.09 - 7.99 (m, 2H), 7.78 (d, *J* = 8.1 Hz, 2H), 7.65 (d, *J =* 7.4 Hz, 2H), 7.58 - 7.53 (m, 1H), 7.49 (t, *J* = 7.9 Hz, 2H), 7.39 (t, *J* = 7.5 Hz, 2H), 7.35 (t, *J* = 7.0 Hz, 1H), 7.31 (d, *J* = 8.2 Hz, 2H), 6.33 (s, 1H), 4.17 (dd, *J* = 18.5, 2.6 Hz, 1H), 3.72 (dd, *J* = 18.5, 2.5 Hz, 1H), 1.92 (s, 1H). |
| PA-12 | 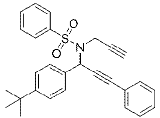 | ¹H NMR (600 MHz, Chloroform-*d*) δ 8.07 - 8.03 (m, 2H), 7.59 (d, *J* = 8.1 Hz, 2H), 7.55 (t, *J* = 7.4 Hz, 1H), 7.49 (t, *J* = 8.0 Hz, 2H), 7.40 (d, *J* = 8.5 Hz, 2H), 7.30 (t, *J* = 7.4 Hz, 1H), 7.28 - 7.24 (m, 2H), 7.20 - 7.16 (m, 2H), 6.28 (s, 1H), 4.15 (dd, *J* = 18.5, 2.6 Hz, 1H), 3.73 (dd, *J =* 18.5, 2.5 Hz, 1H), 1.89 (t, *J* = 2.5 Hz, 1H), 1.32 (s, 9H). |
| PA-13 | 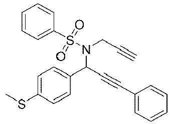 | ¹H NMR (600 MHz, Chloroform-*d*) δ 8.04 (d, *J* = 7.4 Hz, 2H), 7.61 - 7.58 (m, 2H), 7.56 (d, *J =* 14.9 Hz, 1H), 7.49 (t, *J =* 7.7 Hz, 2H), 7.31 (t, *J* = 6.8 Hz, 1H), 7.28 - 7.24 (m, 4H), 7.17 (d, *J =* 7.0 Hz, 2H), 6.26 (s, 1H), 4.12 (dd, *J* = 18.4, 2.5 Hz, 1H), 3.74 (dd, *J* = 18.4, 2.5 Hz, 1H), 2.49 (s, 3H), 1.91 (t, *J* = 2.5 Hz, 1H). |
| PA-14 | 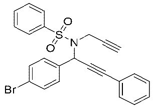 | ¹H NMR (600 MHz, Chloroform-*d*) δ 8.05 - 7.97 (m, 2H), 7.58 - 7.55 (m, 3H), 7.52 - 7.50 (m, 4H), 7.34 - 7.30 (m, 1H), 7.27 (t, *J* = 7.4 Hz, 2H), 7.17 (d, *J* = 7.0 Hz, 2H), 6.25 (s, 1H), 4.10 (dd, *J* = 18.4, 2.5 Hz, 1H), 3.78 (dd, *J* = 18.3, 2.5 Hz, 1H), 1.91 (t, *J* = 2.5 Hz, 1H). |
| PA-15 | 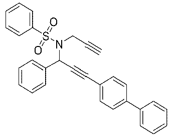 | ¹H NMR (600 MHz, Chloroform-*d*) δ 8.09 - 8.04 (m, 2H), 7.73 - 7.68 (m, 2H), 7.60 - 7.56 (m, 3H), 7.54 - 7.50 (m, 4H), 7.47 - 7.43 (m, 2H), 7.43 - 7.39 (m, 2H), 7.38 - 7.35 (m, 2H), 7.27 (d, *J* = 8.5 Hz, 2H), 6.35 (s, 1H), 4.16 (dd, *J* = 18.4, 2.6 Hz, 1H), 3.78 (dd, *J* = 18.4, 2.5 Hz, 1H), 1.92 (t, *J* = 2.5 Hz, 1H). |
| PA-16 | 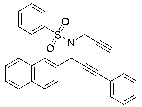 | ¹H NMR (600 MHz, Chloroform-*d*) δ 8.17 (s, 1H), 8.10 - 8.07 (m, 2H), 7.89 - 7.84 (m, 3H), 7.77 - 7.73 (m, 1H), 7.57 (t, *J* = 7.4 Hz, 1H), 7.53 - 7.48 (m, 4H), 7.36 - 7.32 (m, 1H), 7.32 - 7.28 (m, 2H), 7.24 - 7.22 (m, 2H), 6.47 (s, 1H), 4.16 (dd, *J* = 18.5, 2.6 Hz, 1H), 3.76 (dd, *J* = 18.5, 2.5 Hz, 1H), 1.86 (t, *J* = 2.5 Hz, 1H). |
| PA-17 | 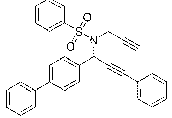 | ¹H NMR (600 MHz, Chloroform-*d*) δ 8.09 - 8.05 (m, 2H), 7.77 - 7.74 (m, 2H), 7.64 - 7.59 (m, 4H), 7.59 - 7.55 (m, 1H), 7.52 - 7.48 (m, 2H), 7.45 (t, *J* = 7.7 Hz, 2H), 7.36 (t, *J* = 7.4 Hz, 1H), 7.34 - 7.30 (m, 1H), 7.28 (t, *J* = 7.3 Hz, 2H), 7.21 (d, *J* = 6.9 Hz, 2H), 6.36 (s, 1H), 4.18 (dd, *J* = 18.4, 2.6 Hz, 1H), 3.80 (dd, *J* = 18.4, 2.5 Hz, 1H), 1.91 (t, *J* = 2.5 Hz, 1H). |
| PA-18 | 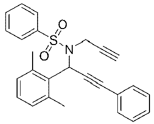 | ¹H NMR (600 MHz, Chloroform-*d*) δ 7.78 - 7.73 (m, 2H), 7.43 (t, *J* = 7.5 Hz, 1H), 7.30 - 7.27 (m, 3H), 7.27 - 7.22 (m, 4H), 7.07 (t, *J* = 7.6 Hz, 1H), 6.93 (d, *J* = 7.5 Hz, 2H), 6.39 (s, 1H), 4.21 (dd, *J* = 10.2, 2.5 Hz, 2H), 2.50 (s, 6H), 2.16 (t, *J* = 2.4 Hz, 1H). |
| PA-19 | 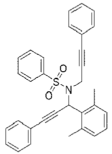 | ¹H NMR (400 MHz, Chloroform-*d*) δ 7.98 (d, *J* = 7.6 Hz, 2H), 7.40 (t, *J* = 7.4 Hz, 1H), 7.30 (t, *J* = 7.8 Hz, 2H), 7.27 - 7.21 (m, 2H), 7.21 - 7.05 (m, 9H), 7.00 (d, *J* = 7.5 Hz, 2H), 6.50 (s, 1H), 4.43 (d, *J* = 18.8 Hz, 1H), 4.28 (d, *J* = 18.8 Hz, 1H), 2.61 (s, 6H). |
| PA-20 | 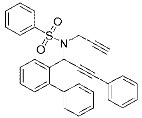 | ¹H NMR (600 MHz, Chloroform-*d*) δ 8.04 - 7.99 (m, 1H), 7.85 - 7.82 (m, 2H), 7.53 (t, *J =* 7.3 Hz, 2H), 7.49 - 7.41 (m, 6H), 7.36 - 7.25 (m, 6H), 7.17 (d, *J =* 6.9 Hz, 2H), 6.40 (s, 1H), 4.08 (dd, *J* = 18.6, 2.5 Hz, 1H), 3.78 (dd, *J* = 18.6, 2.5 Hz, 1H), 1.94 (t, *J* = 2.5 Hz, 1H). |
| PA-21 | 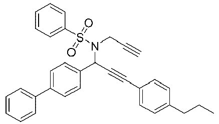 | ¹H NMR (600 MHz, Chloroform-*d*) δ 8.14 - 8.06 (m, 2H), 7.81 (d, *J* = 8.5 Hz, 2H), 7.67 - 7.63 (m, 4H), 7.62 - 7.58 (m, 1H), 7.53 (t, *J* = 7.6 Hz, 2H), 7.48 (t, *J* = 7.7 Hz, 2H), 7.39 (t, *J* = 7.4 Hz, 1H), 7.18 (d, *J* = 8.1 Hz, 2H), 7.13 (d, *J =* 8.0 Hz, 2H), 6.40 (s, 1H), 4.21 (dd, *J* = 18.4, 2.5 Hz, 1H), 3.86 (dd, *J =* 18.4, 2.5 Hz, 1H), 2.63 - 2.58 (m, 2H), 1.96 (t, *J* = 2.5 Hz, 1H), 1.66 (dq, *J* = 14.8, 7.4 Hz, 2H), 0.97 (t, *J* = 7.3 Hz, 3H). |
| PA-22 | 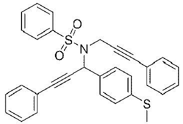 | ¹H NMR (600 MHz, Chloroform-*d*) δ 8.13 - 8.02 (m, 2H), 7.65 (d, *J* = 9.4 Hz, 2H), 7.55 - 7.49 (m, 1H), 7.45 (t, *J* = 7.6 Hz, 2H), 7.27 - 7.25 (m, 3H), 7.22 - 7.18 (m, 1H), 7.17 (t, *J =* 7.8 Hz, 2H), 7.14 - 7.10 (m, 4H), 6.97 - 6.91 (m, 2H), 6.34 (s, 1H), 4.39 (d, *J* = 18.4 Hz, 1H), 4.02 (d, *J* = 18.4 Hz, 1H), 2.45 (s, 3H). |
| PA-23 | 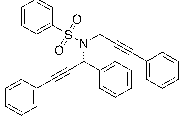 | ¹H NMR (600 MHz, Chloroform-*d*) δ 8.15 - 8.07 (m, 2H), 7.82 - 7.75 (m, 2H), 7.55 - 7.51 (m, 1H), 7.48 - 7.41 (m, 4H), 7.39 - 7.34 (m, 1H), 7.29 - 7.25 (m, 1H), 7.21 (t, *J* = 8.2 Hz, 1H), 7.19 - 7.14 (m, 4H), 7.14 - 7.10 (m, 2H), 6.99 - 6.91 (m, 2H), 6.41 (s, 1H), 4.45 (dd, *J* = 18.5, 4.4 Hz, 1H), 3.99 (dd, *J =* 18.4, 5.4 Hz, 1H). |
| PA-24 | 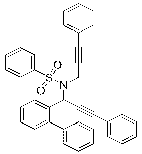 | ¹H NMR (600 MHz, Chloroform-*d*) δ 8.08 - 8.04 (m, 1H), 7.89 - 7.86 (m, 2H), 7.51 (t, *J* = 7.3 Hz, 2H), 7.47 - 7.39 (m, 7H), 7.32 - 7.29 (m, 3H), 7.22 (t, *J* = 7.5 Hz, 2H), 7.14 - 7.11 (m, 4H), 7.05 - 7.00 (m, 4H), 6.40 (s, 1H), 4.31 (d, *J* = 18.7 Hz, 1H), 3.93 (d, *J =* 18.7 Hz, 1H). |
| PA-25 | 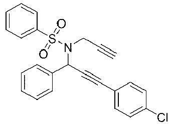 | ¹H NMR (600 MHz, Chloroform-*d*) δ 8.06 - 8.02 (m, 2H), 7.76 - 7.72 (m, 2H), 7.57 - 7.51 (m, 1H), 7.51 - 7.47 (m, 2H), 7.42 - 7.37 (m, 2H), 7.37 - 7.33 (m, 2H), 7.26 - 7.23 (m, 1H), 7.18 - 7.12 (m, 2H), 6.37 (s, 1H), 4.13 (dd, *J =* 18.4, 2.5 Hz, 1H), 3.82 (dd, *J* = 18.4, 2.6 Hz, 1H), 1.87 (t, *J* = 2.5 Hz, 1H). |
| PA-26 | 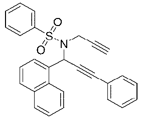 | ¹H NMR (600 MHz, Chloroform-*d*) δ 8.57 (d, *J* = 8.5 Hz, 1H), 8.16 - 8.05 (m, 3H), 7.92 - 7.87 (m, 2H), 7.68 - 7.63 (m, 1H), 7.57 - 7.52 (m, 1H), 7.52 - 7.46 (m, 2H), 7.46 - 7.41 (m, 2H), 7.32 - 7.28 (m, 1H), 7.28 - 7.25 (m, 2H), 7.16 - 7.09 (m, 2H), 7.06 (s, 1H), 4.14 (dd, *J =* 18.7, 2.5 Hz, 1H), 3.52 (dd, *J =* 18.6, 2.5 Hz, 1H), 1.78 (t, *J* = 2.5 Hz, 1H). |
| PA-27 | 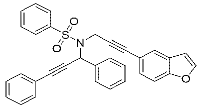 | ¹H NMR (600 MHz, Chloroform-*d*) δ 8.14 - 8.08 (m, 2H), 7.75 (d, *J* = 7.9 Hz, 2H), 7.57 (d, *J* = 2.2 Hz, 1H), 7.49 (t, *J* = 7.4 Hz, 1H), 7.45 - 7.39 (m, 4H), 7.35 (t, *J* = 7.2 Hz, 1H), 7.24 - 7.21 (m, 2H), 7.17 - 7.08 (m, 5H), 6.85 (dd, *J* = 8.5, 1.6 Hz, 1H), 6.56 (dd, *J* = 2.2, 0.9 Hz, 1H), 6.38 (s, 1H), 4.44 (d, *J* = 18.4 Hz, 1H), 3.95 (d, *J =* 18.4 Hz, 1H). |
| PA-28 | 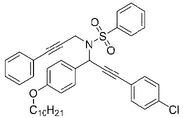 | ¹H NMR (600 MHz, Chloroform-*d*) δ 8.11 - 8.05 (m, 2H), 7.59 (d, *J* = 8.9 Hz, 2H), 7.51 (t, *J* = 8.1 Hz, 1H), 7.44 (t, *J* = 8.0 Hz, 2H), 7.22 (t, *J* = 6.9 Hz, 1H), 7.10 (dd, *J* = 23.9, 8.3 Hz, 4H), 6.98 (d, *J* = 8.6 Hz, 2H), 6.94 - 6.89 (m, 4H), 6.30 (s, 1H), 4.41 (d, *J* = 18.5 Hz, 1H), 3.94 - 3.90 (m, 3H), 1.80 - 1.74 (m, 2H), 1.47 - 1.41 (m, 2H), 1.26 (s, 12H), 0.88 (s, 3H). |
| PA-29 | 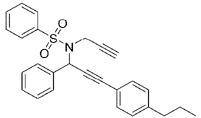 | ¹H NMR (600 MHz, Chloroform-*d*) δ 8.07 - 8.02 (m, 2H), 7.73 - 7.66 (m, 2H), 7.59 - 7.54 (m, 1H), 7.52 - 7.46 (m, 2H), 7.41 - 7.37 (m, 2H), 7.36 - 7.33 (m, 1H), 7.11 (d, *J* = 8.3 Hz, 2H), 7.08 (d, *J* = 8.4 Hz, 2H), 6.31 (s, 1H), 4.12 (dd, *J* = 18.4, 2.6 Hz, 1H), 3.76 (dd, *J* = 18.7, 2.6 Hz, 1H), 2.60 - 2.51 (m, 2H), 1.89 (t, *J* = 2.5 Hz, 1H), 1.66 - 1.60 (m, 2H), 0.92 (t, *J* = 7.3 Hz, 3H). |
| PA-30 | 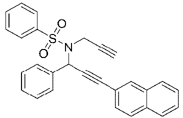 | ¹H NMR (600 MHz, Chloroform-*d*) δ 8.08 (d, *J* = 7.2 Hz, 2H), 7.83 - 7.78 (m, 1H), 7.76 - 7.71 (m, 4H), 7.68 (s, 1H), 7.57 (t, *J* = 7.4 Hz, 1H), 7.53 - 7.48 (m, 4H), 7.41 (t, *J* = 7.4 Hz, 2H), 7.37 (t, *J* = 7.3 Hz, 1H), 7.21 (dd, *J =* 8.4, 1.6 Hz, 1H), 6.37 (s, 1H), 4.18 (dd, *J* = 18.4, 2.5 Hz, 1H), 3.79 (dd, *J=* 18.4, 2.5 Hz, 1H), 1.91 (t, *J* = 2.5 Hz, 1H). |
| PA-31 | 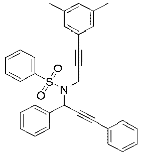 | ¹H NMR (600 MHz, Chloroform-*d*) δ 8.14 - 8.10 (m, 2H), 7.78 - 7.75 (m, 2H), 7.54 - 7.50 (m, 1H), 7.47 - 7.40 (m, 4H), 7.39 - 7.35 (m, 1H), 7.29 - 7.25 (m, 1H), 7.20 - 7.14 (m, 4H), 6.83 (s, 1H), 6.54 (s, 2H), 6.39 (s, 1H), 4.46 (d, *J =* 18.5 Hz, 1H), 3.92 (d, *J* = 18.5 Hz, 1H), 2.13 (s, 6H). |
| PA-32 | 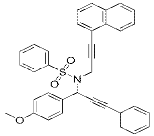 | ¹H NMR (400 MHz, Chloroform-*d*) δ 8.11 (d, *J* = 7.5 Hz, 2H), 7.88 (d, *J* = 8.3 Hz, 1H), 7.77 - 7.65 (m, 4H), 7.48 - 7.27 (m, 6H), 7.26 - 7.10 (m, 4H), 7.08 (d, *J* = 2.6 Hz, 2H), 6.89 (d, *J* = 8.7 Hz, 2H), 6.38 (s, 1H), 4.54 (d, *J* = 18.5 Hz, 1H), 4.17 (d, *J =* 18.5 Hz, 1H), 3.70 (s, 3H). |
| PA-33 | 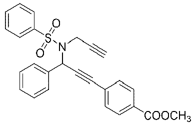 | ¹H NMR (600 MHz, Chloroform-*d*) δ 8.07 - 8.02 (m, 2H), 7.95 - 7.92 (m, 2H), 7.66 (d, *J* = 7.7 Hz, 2H), 7.58 - 7.53 (m, 1H), 7.49 (t, *J* = 7.7 Hz, 2H), 7.40 (t, *J* = 7.2 Hz, 2H), 7.36 (t, *J* = 7.4 Hz, 1H), 7.22 (d, *J* = 8.5 Hz, 2H), 6.33 (s, 1H), 4.17 (dd, *J* = 18.5, 2.5 Hz, 1H), 3.91 (s, 3H), 3.72 (dd, *J* = 18.5, 2.5 Hz, 1H), 1.90 (s, 1H). |
| PA-34 | 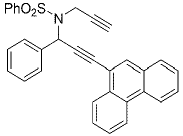 | ¹H NMR (600 MHz, Chloroform-*d*) δ 8.68 - 8.63 (m, 2H), 8.14 - 8.10 (m, 2H), 8.06 (d, *J* = 8.1 Hz, 1H), 7.83 - 7.78 (m, 3H), 7.70 (s, 1H), 7.69 - 7.66 (m, 2H), 7.63 - 7.59 (m, 1H), 7.57 - 7.53 (m, 1H), 7.53 - 7.50 (m, 1H), 7.49 - 7.43 (m, 4H), 7.42 - 7.37 (m, 1H), 6.53 (s, 1H), 4.26 (dd, *J =* 18.5, 2.5 Hz, 1H), 3.85 (dd, *J* = 18.4, 2.5 Hz, 1H), 1.89 (t, *J* = 2.5 Hz, 2H). |
| PA-35 | 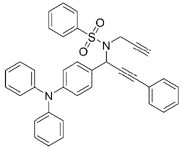 | ¹H NMR (600 MHz, Chloroform-*d*) δ 8.07 (d, *J* = 6.5 Hz, 2H), 7.59 - 7.53 (m, 3H), 7.50 (t, *J* = 7.7 Hz, 2H), 7.33 - 7.29 (m, 1H), 7.29 - 7.25 (m, 6H), 7.19 (d, *J* = 7.9 Hz, 2H), 7.12 - 7.08 (m, 6H), 7.05 (t, *J* = 7.7 Hz, 2H), 6.29 (s, 1H), 4.18 (d, *J* = 18.4 Hz, 1H), 3.87 (d, *J =* 18.4 Hz, 1H), 1.97 (s, 1H). |
| PA-36 | 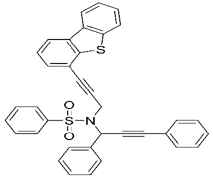 | ¹H NMR (600 MHz, Chloroform-*d*) δ 8.18 - 8.13 (m, 2H), 8.11 - 8.06 (m, 1H), 8.00 (dd, *J* = 7.9, 1.1 Hz, 1H), 7.82 - 7.78 (m, 3H), 7.49 - 7.41 (m, 7H), 7.37 - 7.34 (m, 1H), 7.20 - 7.16 (m, 2H), 7.13 - 7.10 (m, 2H), 7.09 - 7.06 (m, 2H), 6.91 (dd, *J* = 7.4, 1.1 Hz, 1H), 6.42 (s, 1H), 4.58 (d, *J* = 18.4 Hz, 1H), 4.11 (d, *J* = 18.5 Hz, 1H). |
| PA-37 | 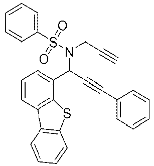 | ¹H NMR (600 MHz, Chloroform-*d*) δ 8.21 - 8.16 (m, 2H), 8.14 - 8.10 (m, 2H), 7.96 (dt, *J* = 7.5, 1.0 Hz, 1H), 7.95 - 7.91 (m, 1H), 7.55 - 7.50 (m, 2H), 7.50 - 7.44 (m, 4H), 7.35 - 7.31 (m, 1H), 7.30 - 7.26 (m, 2H), 7.19 - 7.16 (m, 2H), 6.67 (s, 1H), 4.21 (dd, *J* = 18.9, 2.8 Hz, 1H), 3.64 (dd, *J* = 18.6, 2.5 Hz, 1H), 1.80 (t, *J* = 2.5 Hz, 1H). |
| PA-38 | 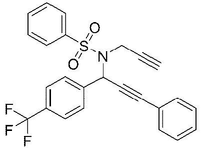 | ¹H NMR (600 MHz, Chloroform-*d*) δ 8.04 (d, *J* = 7.2 Hz, 2H), 7.85 (d, *J* = 8.7 Hz, 2H), 7.66 (d, *J* = 8.2 Hz, 2H), 7.62 - 7.58 (m, 1H), 7.55 - 7.50 (m, 2H), 7.37 - 7.32 (m, 1H), 7.32 - 7.28 (m, 2H), 7.23 - 7.19 (m, 2H), 6.36 (s, 1H), 4.13 (dd, *J* = 18.3, 2.5 Hz, 1H), 3.84 (dd, *J* = 18.3, 2.6 Hz, 1H), 1.98 - 1.87 (m, 1H). |
| PA-39 | 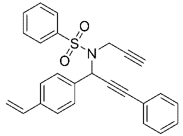 | ¹H NMR (600 MHz, Chloroform-*d*) δ 8.08 - 8.04 (m, 2H), 7.66 (d, *J* = 8.4 Hz, 2H), 7.59 - 7.54 (m, 1H), 7.52 - 7.48 (m, 2H), 7.43 (d, *J* = 8.3 Hz, 2H), 7.34 - 7.30 (m, 1H), 7.29 - 7.26 (m, 2H), 7.22 - 7.18 (m, 2H), 6.73 (dd, *J* = 17.6, 10.9 Hz, 1H), 6.31 (s, 1H), 5.79 (dd, *J* = 17.6, 0.8 Hz, 1H), 5.35 - 5.20 (m, 1H), 4.15 (dd, *J* = 18.7, 2.8 Hz, 1H), 3.78 (dd, *J* = 18.5, 2.5 Hz, 1H), 1.92 (t, *J* = 2.5 Hz, 1H). |
| PA-40 | 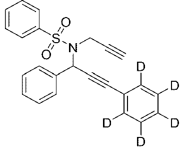 | ¹H NMR (400 MHz, Chloroform-*d*) δ 7.96 (d, *J* = 7.5 Hz, 2H), 7.61 (d, *J =* 7.3 Hz, 2H), 7.46 (t, *J =* 7.3 Hz, 1H), 7.39 (t, *J* = 7.5 Hz, 2H), 7.27 (dd, *J =* 11.3, 7.2 Hz, 3H), 6.23 (s, 1H), 4.05 (dd, *J* = 18.4, 2.3 Hz, 1H), 3.67 (dd, *J* = 18.4, 2.4 Hz, 1H), 1.81 (t, *J* = 2.4 Hz, 1H). |

### Example 2 Preparation of IM-1

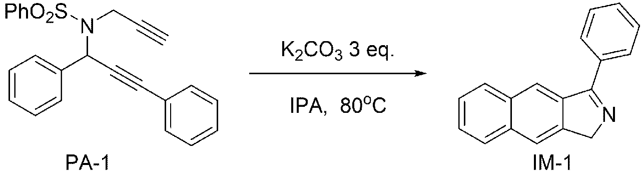

The compound PA-1 (0.5 mmol) and potassium carbonate (1.5 mmol) are added to a reaction tube, and 2 mL of isopropanol is added as a solvent. After reacting under nitrogen at 80°C for 24 hours, the reaction solution is spin-dried and passed through a column to obtain the compound IM-1. The entire reaction and post-processing process must be kept in an oxygen-free state (yield 70%).

The NMR data of the product are as follows:
¹H NMR (600 MHz, CDCl₃) δ 8.29 (s, 1H), 8.10 - 8.01 (m, 3H), 7.99 (d, *J* = 8.2 Hz, 1H), 7.94 (d, *J =* 8.2 Hz, 1H), 7.61 - 7.57 (m, 4H), 7.55 - 7.52 (m, 1H), 5.14 (s, 2H). ¹³C NMR (151 MHz, CDCl₃) δ 172.35, 146.57, 137.23, 134.91, 133.21, 132.73, 130.25, 129.37, 128.88, 128.33, 128.11, 127.26, 125.98, 121.98, 120.83, 63.91.

The high-resolution mass spectrometry data of the product are as follows:
HRMS *m*/*z* (ESI) calcd for [C₁₈H₁₃NH] ([M+H]⁺): 244.1121, found: 244.1121.

### Example 3 Preparation of IM-2

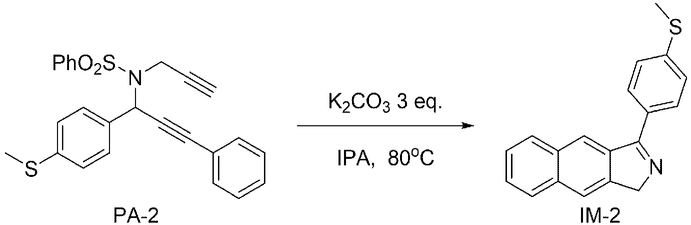

The compound PA-2 (0.5 mmol) and potassium carbonate (1.5 mmol) are added to a reaction tube, and 2 mL of isopropanol is added as a solvent. After reacting for 24 hours under argon at 80 °C , the reaction solution is spin-dried and passed through a column to obtain compound IM-2. The entire reaction and post-processing process must be kept in an oxygen-free state (yield 48%).

The NMR data of the product are as follows:
¹H NMR (600 MHz, Chloroform-*d*) δ 8.27 (s, 1H), 8.04 (s, 1H), 7.98 (d, *J* = 8.4 Hz, 3H), 7.93 (d, *J =* 8.2 Hz, 1H), 7.59 - 7.56 (m, 1H), 7.54 - 7.51 (m, 1H), 7.44 - 7.42 (m, 2H), 2.57 (s, 3H).

### Example 4 Preparation of IM-1

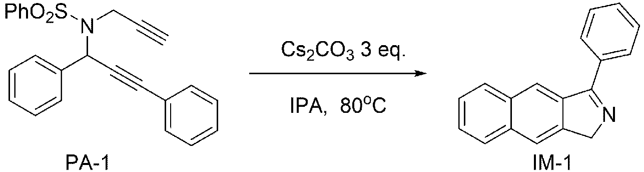

The compound PA-1 (0.5 mmol) and cesium carbonate (1.5 mmol) are added to a reaction tube, and 2 mL of isopropanol is added as a solvent. After reacting under argon at 80 °C for 24 hours, the reaction solution is spin-dried and passed through a column to obtain compound IM-1. The entire reaction and post-processing process must be kept in an oxygen-free state (yield 62%).

The NMR data of the product are as follows:
¹H NMR (600 MHz, CDCl₃) δ 8.29 (s, 1H), 8.10 - 8.01 (m, 3H), 7.99 (d, *J* = 8.2 Hz, 1H), 7.94 (d, *J =* 8.2 Hz, 1H), 7.61 - 7.57 (m, 4H), 7.55 - 7.52 (m, 1H), 5.14 (s, 2H). ¹³C NMR (151 MHz, CDCl3) δ 172.35, 146.57, 137.23, 134.91, 133.21, 132.73, 130.25, 129.37, 128.88, 128.33, 128.11, 127.26, 125.98, 121.98, 120.83, 63.91.

The high-resolution mass spectrometry data of the product are as follows:
HRMS *m*/*z* (ESI) calcd for [C₁₈H₁₃NH] ([M+H]⁺): 244.1121, found: 244.1121.

### Example 5 Preparation of IM-1

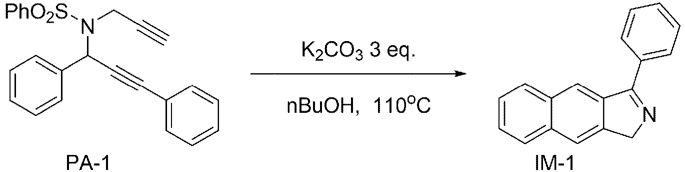

The compound PA-1 (0.5 mmol) and potassium carbonate (1.5 mmol) are added to a reaction tube, and 2 mL of n-butanol is added as a solvent. After reacting under nitrogen at 120 °C for 24 hours, the reaction solution is spin-dried and passed through a column to obtain compound IM-1. The entire reaction and post-processing process must be kept in an oxygen-free state (yield 82%).

The NMR data of the product are as follows:
¹H NMR (600 MHz, CDCl₃) δ 8.29 (s, 1H), 8.10 - 8.01 (m, 3H), 7.99 (d, *J* = 8.2 Hz, 1H), 7.94 (d, *J =* 8.2 Hz, 1H), 7.61 - 7.57 (m, 4H), 7.55 - 7.52 (m, 1H), 5.14 (s, 2H). ¹³C NMR (151 MHz, CDCl₃) δ 172.35, 146.57, 137.23, 134.91, 133.21, 132.73, 130.25, 129.37, 128.88, 128.33, 128.11, 127.26, 125.98, 121.98, 120.83, 63.91.

The high-resolution mass spectrometry data of the product are as follows:
HRMS *m*/*z* (ESI) calcd for [C₁₈H₁₃NH] ([M+H]⁺): 244.1121, found: 244.1121.

### Example 6 Preparation of IM-2

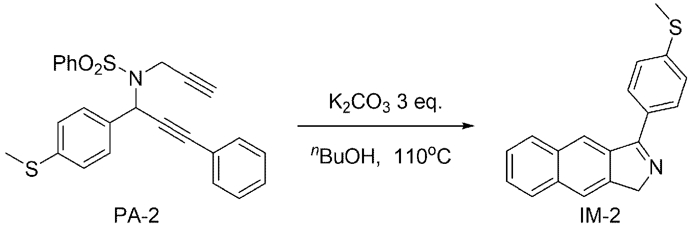

Compound PA-2 (0.5 mmol) and potassium carbonate (1.5 mmol) are added to a reaction tube, and 2 mL of isopropanol is added as a solvent. After reacting under nitrogen at 80 °C for 24 hours, the reaction solution is spin-dried and passed through a column to obtain compound IM-2. The entire reaction and post-processing process must be kept in an oxygen-free state (yield 78%).

The NMR data of the product are as follows:
¹H NMR (600 MHz, Chloroform-*d*) δ 8.27 (s, 1H), 8.04 (s, 1H), 7.98 (d, *J* = 8.4 Hz, 3H), 7.93 (d, *J =* 8.2 Hz, 1H), 7.59 - 7.56 (m, 1H), 7.54 - 7.51 (m, 1H), 7.44 - 7.42 (m, 2H), 2.57 (s, 3H).

### Example 7 Preparation of dimer compound by IM-1 converting

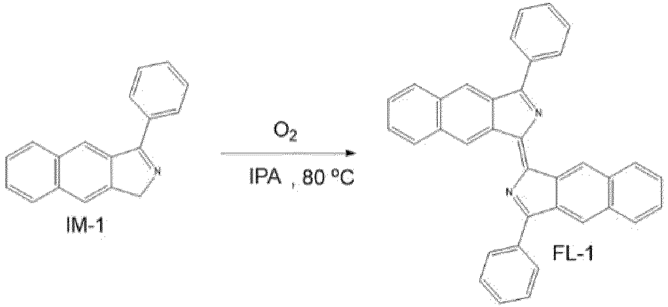

The compound IM-1 (0.2 mmol) is dissolved in 1 mL of isopropanol solvent, oxygen in the air is used as an oxidant, and the mixture is reacted at 80 °C for 24 hours. After the reaction is completed, the reaction solution is spin-dried and passed through a column to obtain the dimer compound FL-1. (Yield 38%).

The NMR data of the product FL-1 are as follows:
1H NMR (400 MHz, CDCl₃) δ 9.49 (s, 2H), 8.45 - 8.40 (m, 4H), 8.36 (s, 2H), 8.12 (d, *J*= 7.8 Hz, 2H), 7.99 (d, *J* = 7.8 Hz, 2H), 7.73 - 7.65 (m, 6H), 7.62 - 7.55 (m, 4H).
¹³C NMR (150 MHz, CDCl₃) δ 170.75, 148.39, 137.13, 135.36, 134.99, 133.73, 133.16, 131.12, 130.06, 129.90, 129.26, 129.06, 127.86, 127.49, 126.96, 122.98.

The high-resolution mass spectrometry data of the product are as follows:
HRMS *m*/*z* (ESI) calcd for [C₃₆H₂₂N₂H] ([M+H]⁺): 483.1856, found: 483.1855.

### Example 8 Preparation of dimer compound by IM-2 converting

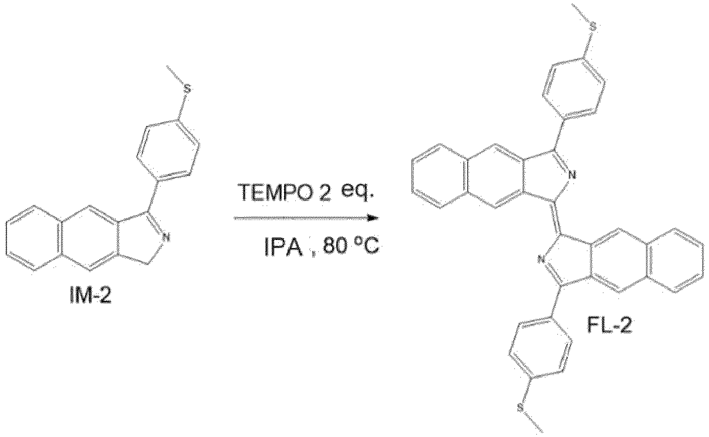

The compound IM-2 (0.2 mmol) is dissolved in 1 mL of isopropanol solvent, and 2,2,6,6-tetramethylpiperidinium N-oxide (TEMPO, 2 equiv.) is added and reacted at 80°C for 24 hours. After the reaction is completed, the reaction solution is spin-dried and passed through a column to obtain the dimer compound FL-2. (Yield 18%).

The NMR data of the product FL-2 are as follows:
¹H NMR (400 MHz, CDCl₃/CF₃COOD, 5:1) δ 9.54 (s, 2H), 9.07 (s, 2H), 8.31 (d, *J* = 8.3 Hz, 4H), 8.26 (d, *J* = 8.2 Hz, 4H), 8.02 (t, *J* = 7.5 Hz, 2H), 7.95 (t, *J* = 7.5 Hz, 2H), 7.64 (d, *J* = 8.1 Hz, 4H), 2.71 (s, 6H).
¹³C NMR (150 MHz, CDCl₃/CF₃COOD, 5:1) δ 166.84, 156.87, 136.41, 134.55, 134.14, 133.21, 131.97, 131.70, 131.49, 131.12, 130.76, 128.61, 128.23, 128.01, 126.65, 120.88, 14.57.

The high-resolution mass spectrometry data of the product FL-2 are as follows:
HRMS *m*/*z* (ESI) calcd for [C₃₈H₂₆N₂S₂Na] ([M+Na]⁺): 597.1430, found: 597.1427.

### Example 9 Preparation of dimer compound by IM-3 converting

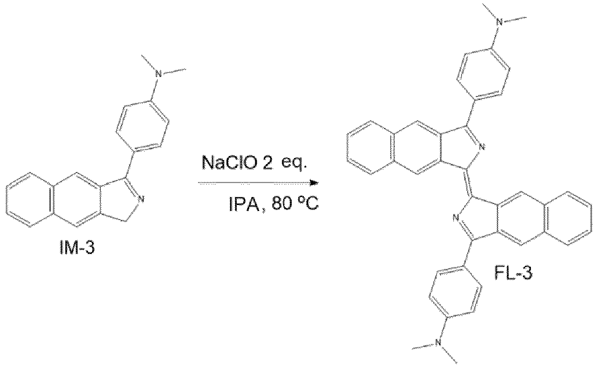

The compound IM-3 (0.2 mmol) is dissolved in 1 mL of isopropanol solvent, and 8% sodium hypochlorite aqueous solution (2 equiv.) is added, and the mixture is reacted at 80°C for 24 hours. After the reaction is completed, the reaction solution is spin-dried and passed through a column to obtain the dimer compound FL-3. (Yield 68%).

The NMR data of the product FL-3 are as follows:
¹H NMR (600 MHz, CDCl₃/CF₃COOD, 5:1) δ 9.50 (s, 2H), 9.00 (s, 2H), 8.43 (d, *J* = 8.8 Hz, 4H), 8.26 (d, *J* = 8.3 Hz, 4H), 7.96 (t, *J* = 7.6 Hz, 2H), 7.90 (t, *J* = 7.5 Hz, 2H), 7.59 (d, *J* = 8.8 Hz, 4H), 3.48 (s, 12H).
¹³C NMR (150 MHz, CDCl₃/CF₃COOD, 5:1) δ 159.69, 152.52, 136.01, 134.37, 134.23, 133.89, 132.47, 132.32, 131.42, 131.21, 131.01, 130.24, 128.93, 128.18, 127.01, 119.17, 44.11. IR (thin film) v 1433, 1455, 1598, 2850, 2919 cm⁻¹.

The high-resolution mass spectrometry data of the product FL-3 are as follows:
HRMS *m*/*z* (ESI) calcd for [C₄₀H₃₂N₄H] ([M+H]⁺): 569.2700, found: 569.2708.

### Example 10 Preparation of dimer compound by IM-4 converting

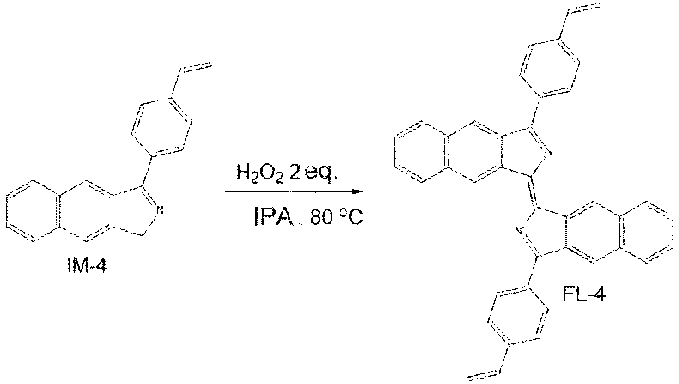

The compound IM-4 (0.2 mmol) is dissolved in 1 mL of isopropanol solvent, and a 30% aqueous hydrogen peroxide solution (2 equiv.) is added and reacted at 80°C for 24 hours. After the reaction is completed, the reaction solution is spin-dried and passed through a column to obtain the dimer compound FL-4. (Yield 22%).

The NMR data of the product FL-4 are as follows:
¹¹H NMR (600 MHz, CDCl₃) δ 9.47 (s, 2H), 8.41 (d, *J* = 8.2 Hz, 4H), 8.37 (s, 2H), 8.11 (d, *J* = 8.0 Hz, 2H), 7.98 (d, *J* = 7.9 Hz, 2H), 7.71 (d, *J* = 8.1 Hz, 4H), 7.59 (t, *J* = 7.2 Hz, 2H), 7.55 (t, *J* = 7.9 Hz, 2H), 6.88 (dd, *J* = 17.6, 10.8 Hz, 2H), 5.97 (d, *J* = 17.6 Hz, 2H), 5.43 (d, *J* = 10.9 Hz, 2H).
¹³C NMR (150 MHz, CDCl₃) δ 169.99, 148.39, 140.24, 137.18, 136.51, 135.35, 134.35, 133.69, 133.13, 130.03, 129.89, 129.48, 127.78, 127.49, 126.96, 126.85, 122.95, 115.76. IR (thin film) v 1474, 1500, 1603, 1623, 2849, 2918 cm⁻¹.

The high-resolution mass spectrometry data of the product FL-4 are as follows:
HRMS *m*/*z* (ESI) calcd for [C₄₀H₂₆N₂H] ([M+H]⁺): 535.2169, found: 535.2169.

### Example 11 Preparation of dimer compound by IM-5 converting

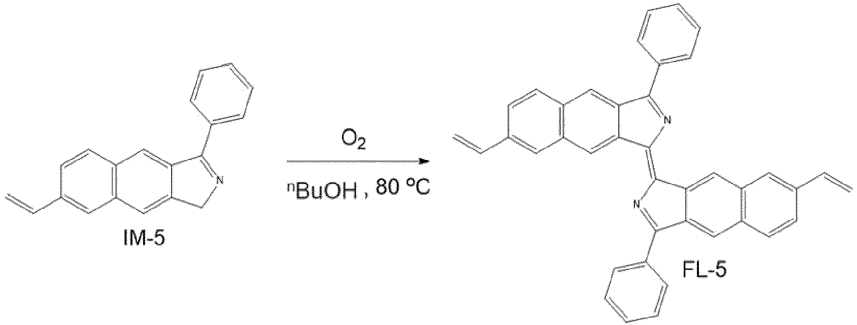

The compound IM-5 (0.2 mmol) is dissolved in 1 mL of isopropanol solvent and reacted at 80 °C for 24 hours in an atmosphere of oxygen in air as an oxidant. After the reaction is completed, the reaction solution is spin-dried and passed through a column to obtain the dimer compound FL-5. (Yield 22%).

The NMR data of the product FL-5 are as follows:
¹H NMR (400 MHz, CDCl₃) δ 9.37 (s, 2H), 8.39 (d, *J* = 7.6 Hz, 4H), 8.18 (s, 2H), 7.94 (s, 2H), 7.88 (d, *J* = 8.5 Hz, 2H), 7.71 - 7.66 (m, 8H), 6.94 (dd, *J* = 17.6, 10.9 Hz, 2H), 5.96 (d, *J* = 17.6 Hz, 2H), 5.43 (d, *J* = 10.8 Hz, 2H).
¹³C NMR (150 MHz, CDCl₃) δ 170.61, 148.42, 137.55, 136.89, 136.59, 135.29, 134.97, 133.83, 132.74, 131.07, 130.07, 129.27, 129.02, 128.49, 127.96, 124.17, 122.64, 115.04. IR (thin film) v 1445, 1473, 2980, 3006, 3050 cm⁻¹.

The high-resolution mass spectrometry data of the product FL-5 are as follows:
HRMS m/z (ESI) calcd for [C₄₀H₂₆N₂H] ([M+H]⁺): 535.2169, found: 535.2169.

### Example 12 Preparation of dimer compound by IM-6 converting

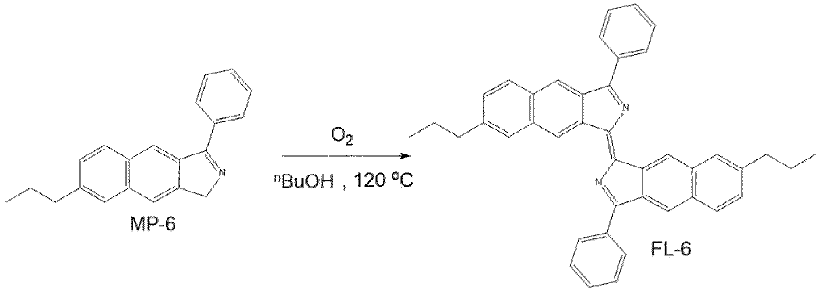

The compound IM-6 (0.2 mmol) is dissolved in 1 mL of isopropanol solvent and reacted under an oxygen atmosphere at 120°C for 24 hours. After the reaction is completed, the reaction solution is spin-dried and passed through a column to obtain the dimer compound FL-6. (Yield 42%).

The NMR data of the product FL-6 are as follows:
¹H NMR (400 MHz, CDCl₃) δ 9.42 (s, 2H), 8.43 (d, *J* = 7.2 Hz, 4H), 8.31 (s, 2H), 7.95 - 7.86 (m, 4H), 7.73 - 7.62 (m, 6H), 7.41 (d, *J=* 8.2 Hz, 2H), 2.83 (t, *J =* 7.6 Hz, 4H), 1.87 - 1.73 (m, 4H), 1.03 (t, *J* = 7.3 Hz, 6H).
¹³C NMR (150 MHz, CDCl₃) δ 170.79, 148.44, 142.25, 137.30, 135.13, 134.78, 134.00, 131.63, 131.00, 129.71, 129.24, 129.03, 128.69, 128.66, 127.47, 122.77, 38.40, 24.54, 14.00.

The high-resolution mass spectrometry data of the product FL-6 are as follows:
HRMS *m*/*z* (ESI) calcd for [C₄₂H₃₄N₂H] ([M+H]⁺): 567.2795, found: 567.2795.

### Example 13 Preparation of dimer compound by IM-7 converting

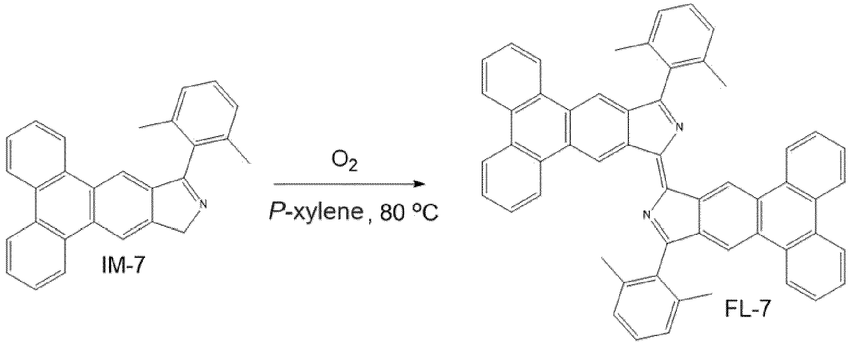

The compound IM-7 (0.2 mmol) is dissolved in 1 mL of p-xylene solvent and reacted under an oxygen atmosphere at 80 °C for 24 hours. After the reaction is completed, the reaction solution is spin-dried and passed through a column to obtain the dimer compound FL-7. (Yield 33%).

The NMR data of the product FL-7 are as follows:
¹H NMR (400 MHz, CDCl₃/CF₃COOD, 5:1) δ 10.30 (s, 2H), 8.86 (d, *J* = 8.1 Hz, 2H), 8.73 (s, 2H), 8.71 - 8.65 (m, 4H), 8.54 (d, *J* = 8.1 Hz, 2H), 7.80 - 7.71 (m, 6H), 7.68 (t, *J* = 7.5 Hz, 2H), 7.58 (t, *J* = 7.7 Hz, 2H), 7.42 (d, *J* = 7.7 Hz, 4H), 2.45 (s, 12H).
¹³C NMR (100 MHz, CDCl₃/CF₃COOD, 5:1) δ 177.99, 137.03, 134.76, 134.18, 133.70, 132.22, 131.19, 130.97, 129.55, 129.19, 129.10, 129.03, 128.53, 128.34, 127.87, 125.06, 124.86, 123.84, 123.71, 123.47, 121.42, 20.55.

The high-resolution mass spectrometry data of the product FL-7 are as follows:
HRMS *m*/*z* (ESI) calcd for [C₅₆H₃₈N₂H] ([M+H]⁺): 739.3108, found: 739.3108.

### Example 14 Preparation of a heterodimeric compound by IM-1 and IM-8 converting

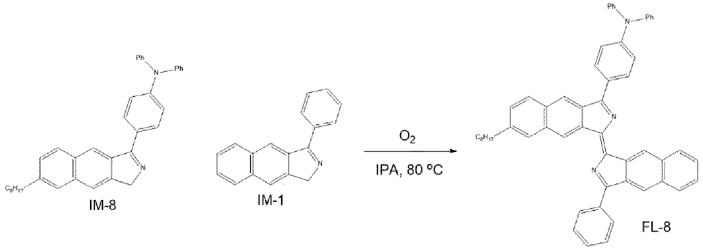

The compound IM-8 (0.1 mmol) and IM-1 (0.1 mmol) are dissolved in 1 mL of isopropanol solvent and reacted under an oxygen atmosphere at 80 °C for 24 hours. After the reaction is completed, the reaction solution is spin-dried and passed through a column to obtain the heterodimeric compound FL-8. (Yield 21%).

The NMR data of the product FL-8 are as follows:
¹H NMR (600 MHz, CDCl₃) δ 9.46 (s, 1H), 9.42 (s, 1H), 8.43 - 8.41 (m, 2H), 8.37 - 8.33 (m, 4H), 8.09 (d, *J* = 7.3 Hz, 1H), 7.97 (d, *J* = 8.3 Hz, 1H), 7.88 - 7.84 (m, 2H), 7.70 - 7.67 (m, 2H), 7.65 - 7.61 (m, 1H), 7.57 - 7.51 (m, 2H), 7.40 - 7.35 (m, 5H), 7.29 - 7.25 (m, 6H), 7.18 - 7.14 (m, 2H), 2.84 - 2.80 (m, 2H), 1.77 - 1.72 (m, 2H), 1.40 - 1.27 (m, 10H), 0.87 (t, *J=* 7.1 Hz, 3H).
¹³C NMR (150 MHz, CDCl₃) δ 169.83, 169.57, 150.80, 149.01, 147.19, 147.05, 142.45, 137.52, 137.30, 135.31, 135.22, 134.86, 133.93, 133.65, 133.01, 131.60, 130.82, 130.46, 130.00, 129.83, 129.68, 129.20, 129.01, 128.59, 128.57, 127.88, 127.44, 127.36, 127.27, 126.71, 125.78, 124.31, 122.87, 122.68, 121.69, 36.38, 32.00, 31.50, 29.65, 29.52, 29.40, 22.78, 14.23.

The high-resolution mass spectrometry data of the product FL-8 are as follows:
HRMS *m*/*z* (ESI) calcd for [C₅₆H₄₇N₃H] ([M+H]⁺): 762.3843, found: 762.3846.

### Example 15 Preparation of dimer compound by IM-9 converting

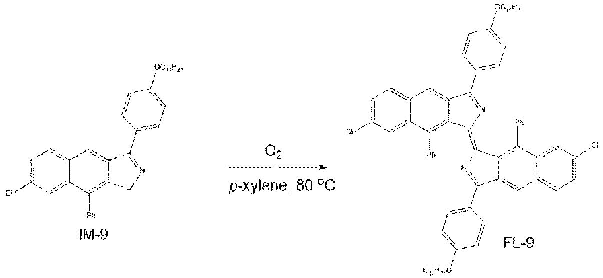

The compound IM-9 (0.2 mmol) is dissolved in 1 mL of p-xylene solvent and reacted under an oxygen atmosphere at 80 °C for 24 hours. After the reaction is completed, the reaction solution is spin-dried and passed through a column to obtain the dimer compound FL-9. (Yield 52%).

The NMR data of the product FL-9 are as follows:
¹H NMR (600 MHz, CDCl₃) δ 8.16 (s, 2H), 8.01 (s, 2H), 7.86 (d, *J =* 8.7 Hz, 2H), 7.78 (d, *J* = 8.8 Hz, 4H), 7.67 - 7.62 (m, 4H), 7.47 - 7.42 (m, 6H), 7.27 (t, *J* = 8.2 Hz, 2H), 6.99 (d, *J* = 8.8 Hz, 4H), 4.06 (t, *J =* 6.6 Hz, 4H), 1.87 - 1.82 (m, 4H), 1.52 - 1.48 (m, 4H), 1.39 - 1.29 (m, 24H), 0.89 (t, *J* = 7.0 Hz, 6H).
¹³C NMR (151 MHz, CDCl₃) δ 164.46, 161.29, 150.00, 139.80, 138.12, 136.77, 136.04, 133.64, 133.34, 131.79, 131.31, 131.02, 130.94, 128.60, 127.54, 127.21, 126.85, 126.47, 122.70, 114.33, 68.30, 32.01, 29.70, 29.68, 29.52, 29.44, 29.33, 26.15, 22.79, 14.24.

The high-resolution mass spectrometry data of the product FL-9 are as follows:
HRMS *m*/*z* (ESI) calcd for [C₆₈H₆₈Cl₂N₂O₂Na] ([M+Na]⁺): 1037.4550, found: 1037.4550.

### Example 16 Fluorescent labeling of L292 cells by IM-1

Cell line: L929 cells (mouse fibroblasts).

Cell culture: Cells are cultured in a CO₂ incubator (37 °C, 5% CO₂) with DMEM medium (penicillin (100 U/mL) and streptomycin (100 µg/mL)).

Cell imaging experiment: Cells are cultured on 15 mm grade bottom cell culture dishes. Before labeling the cells with the probe, the culture medium is removed, and the cells are washed twice with Hank's buffer solution to remove the remaining culture medium. Then, 200 µL of probe IM-1 (10 µM) is added to the culture dish, and the cells are incubated in the dark for 30 minutes. The cells are washed twice with Hank's buffer solution to remove any remaining probe, and then 1 mL of Hank's solution is added to the culture dish. The cells are observed under a laser confocal microscope with a 100x objective. The excitation wavelength is 543 nm, and the emission wavelength is 550-600 nm. It was found that the probe labels the lysosomes and lysosomal autolysosomes in the cells, indicating that this compound can fluorescently label lysosomes and lysosomal autolysosomes in cells.

### Example 17 Fluorescent labeling of L292 cells by IM-2

Cell line: L929 cells (mouse fibroblasts).

Cell culture: Cells are cultured in a CO₂ incubator (37 °C, 5% CO₂) with DMEM medium (penicillin (100 U/mL) and streptomycin (100 µg/mL)).

Cell imaging experiment: Cells are cultured on 15 mm grade bottom cell culture dishes. Before labeling the cells with the probe, the culture medium is removed, and the cells are washed twice with Hank's buffer solution to remove the remaining culture medium. Then, 200 µL of probe IM-2 (5 µM) is added to the culture dish, and the cells are incubated in the dark for 30 minutes. The cells are washed twice with Hank's buffer solution to remove any remaining probe, and then 1 mL of Hank's solution is added to the culture dish. The cells are observed under a laser confocal microscope with a 100x objective. The excitation wavelength is 543 nm, and the emission wavelength is 550-600 nm. It was found that the probe labels the lysosomes and lysosomal autolysosomes in the cells, indicating that this compound can fluorescently label lysosomes and lysosomal autolysosomes in cells.

### Example 18 Fluorescent labeling of L292 cells by IM-3

Cell line: L929 cells (mouse fibroblasts).

Cell culture: Cells are cultured in a CO₂ incubator (37 °C, 5% CO₂) with DMEM medium (penicillin (100 U/mL) and streptomycin (100 µg/mL)).

Cell imaging experiment: Cells are cultured on 15 mm grade bottom cell culture dishes. Before labeling the cells with the probe, the culture medium is removed, and the cells are washed twice with Hank's buffer solution to remove the remaining culture medium. Then, 200 µL of probe IM-3 (2 µM) is added to the culture dish, and the cells are incubated in the dark for 30 minutes. The cells are washed twice with Hank's buffer solution to remove any remaining probe, and then 1 mL of Hank's solution is added to the culture dish. The cells are observed under a laser confocal microscope with a 100x objective. The excitation wavelength is 488 nm, and the emission wavelength is 600-650 nm. It was found that the probe labels the lysosomes and lysosomal autolysosomes in the cells, indicating that this compound can fluorescently label lysosomes and lysosomal autolysosomes in cells.

### Example 19 Testing the fluorescence effect of MP-1 probe molecules on L292 cell mitochondria

Cell line: L929 cells (mouse fibroblasts).

Cell culture: Cells are cultured in a CO₂ incubator (37 °C, 5% CO₂) with DMEM medium (penicillin (100 U/mL) and streptomycin (100 µg/mL)).

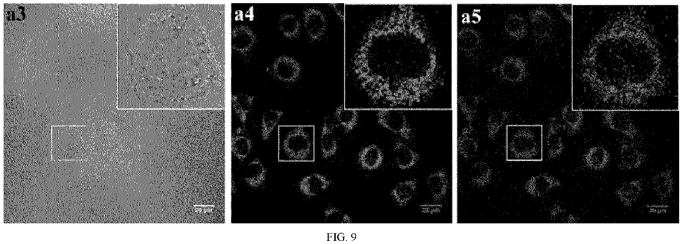

Cell imaging experiment: Cells are cultured on 15 mm grade bottom cell culture dishes. Before labeling the cells with the probe, the culture medium is removed, and the cells are washed twice with Hank's buffer solution to remove the remaining culture medium. Then, 200 µL of probe MP-1 (10 µM) is added to the culture dish, and the cells are incubated in the dark for 30 minutes. The cells are washed twice with Hank's buffer solution to remove any remaining probe, and then 1 mL of Hank's solution is added to the culture dish. The cells are observed under a laser confocal microscope with a 100x objective. The excitation wavelength is 543 nm, and the emission wavelength is 550-600 nm. As shown in FIG. 9.

### Example 20 Testing the fluorescence effect of MP-2 probe molecules on L292 cell mitochondria

Cell line: L929 cells (mouse fibroblasts).

Cell culture: Cells are cultured in a CO₂ incubator (37 °C, 5% CO₂) with DMEM medium (penicillin (100 U/mL) and streptomycin (100 µg/mL)).

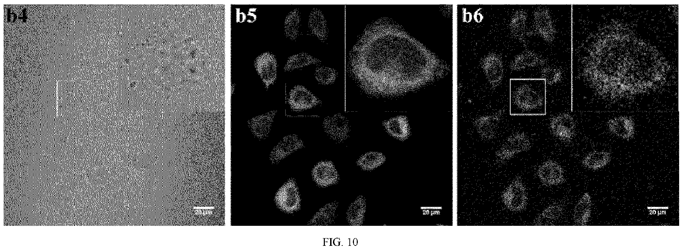

Cell imaging experiment: Cells are cultured on 15 mm grade bottom cell culture dishes. Before labeling the cells with the probe, the culture medium is removed, and the cells are washed twice with Hank's buffer solution to remove the remaining culture medium. Then, 200 µL of probe MP-2 (20 µM) is added to the culture dish, and the cells are incubated in the dark for 30 minutes. The cells are washed twice with Hank's buffer solution to remove any remaining probe, and then 1 mL of Hank's solution is added to the culture dish. The cells are observed under a laser confocal microscope with a 100x objective. The excitation wavelength is 488 nm, and the emission wavelength is 500-540 nm. As shown in FIG. 10.

### Example 21 Testing the fluorescence effect of MP-1 probe molecules on other cell mitochondria

Cell line: RAW 264.7 cell (mouse mononuclear macrophage leukemia cell); SKBR3 cell (HER2-breast cancer overexpressing cell); HuH-7 cell (human hepatoma cell line).

Cell culture: Cells are cultured in a CO₂ incubator (37 °C, 5% CO₂) with DMEM medium (penicillin (100 U/mL) and streptomycin (100 µg/mL)).

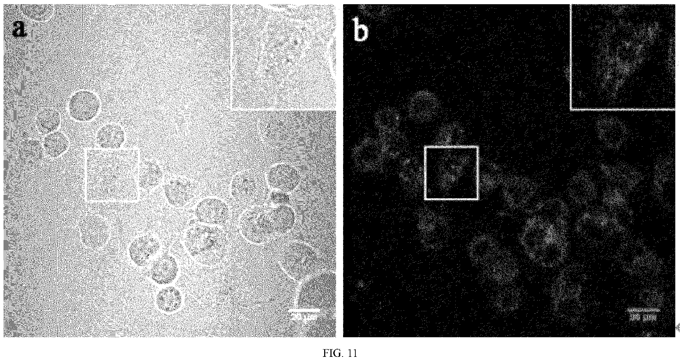

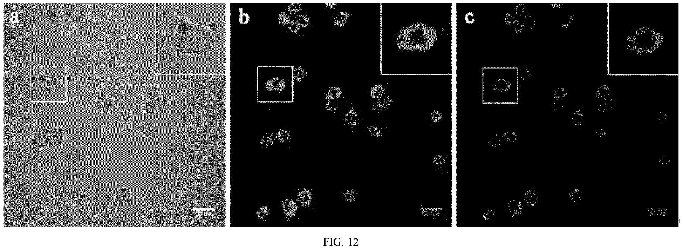

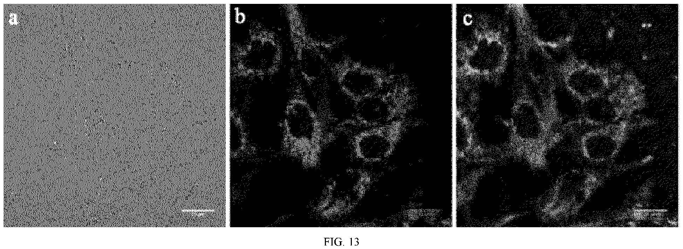

Cell imaging experiment: three types of cells are cultured on 15 mm grade bottom cell culture dishes. Before labeling the cells with the probe, the culture medium is removed, and the cells are washed twice with Hank's buffer solution to remove the remaining culture medium. Then, 200 µL of probe MP-1 (10 µM) is added to the culture dish, and the cells are incubated in the dark for 30 minutes. The cells are washed twice with Hank's buffer solution to remove any remaining probe, and then 1 mL of Hank's solution is added to the culture dish. The cells are observed under a laser confocal microscope with a 100x objective. As shown in FIGS. 11, 12, and 13, the MP-2 probe labels the mitochondria of L929 cells well, with an excitation wavelength of 543 nm, and an emission wavelength of 550 nm-600 nm. This experiment demonstrates that the fluorescent probe of the present application has good adaptability in most microenvironments.

### Example 22 Cell Silencing experiment 1

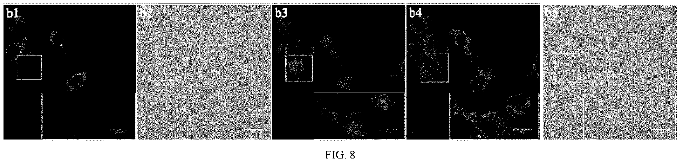

Cells are cultured in imaging culture dishes for 6 h and then treated with cytc-290 (designed silent siRNA) or nontargeting RNA. After a certain period of action, live cell imaging is performed under a confocal laser scanning microscope. After 12 hours of ctyc-290 treatment, Hoechst 33342 and MTG staining are good, but about 10% of the cells could not be labeled by MP-1. About 30% of the cells could not be labeled by MP-1 after 18 hours of treatment with ctyc-290. As shown in FIG. 8, in contrast, the staining effect of MP-1 in the control group using non-targeted RNA is still good. The results show that the probe MP-1 can effectively reflect the activity of the respiratory chain and can be used to detect the absence of enzymes in the respiratory chain.

### Example 23 Cell Silencing experiment 2

The cells are cultured in the imaging culture dish for 6 h, and then treated with cytc-436 (designed silent siRNA) or non-targeting RNA for a certain period of time, and then live cell imaging is performed under a confocal laser scanning microscope. After 6 hours of ctyc-436 treatment, Hoechst 33342 and MTG staining are good, but about 5% of the cells could not be labeled by MP-1. In contrast, the staining effect of MP-1 in the control group using non-targeting RNA is still good. The results show that the probe MP-1 can effectively reflect the activity of the respiratory chain and can be used to detect the absence of enzymes in the respiratory chain.

### Mass spectrometry analysis

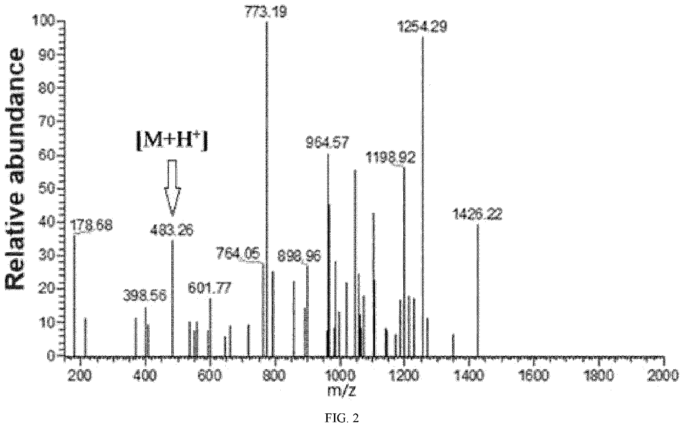

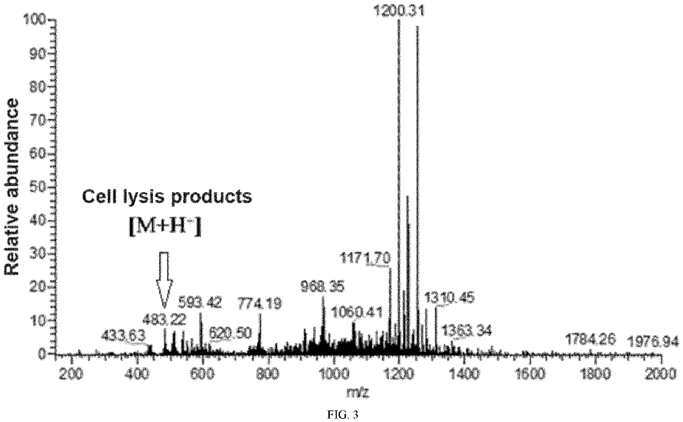

Take the L292 cells of Example 19, remove all the culture medium, add 8 ml of deionized water, and allow the cells to absorb water and burst for about 1 hour. The broken liquid is then collected, ultrasonicated at 20 kHz for 10 minutes, vortexed at 2500 rpm for 10 minutes, and finally extracted with dichloromethane. The organic phase is taken out and then 1/1000 trifluoroacetic acid is added for mass spectrometry analysis. From FIG. 2, the molecular weight of the benzindole dimer is 483.26 (C₃₆H₂₃N₂⁺), and from FIG. 3, the molecular weight obtained in the cell lysate is 483.22. The experimental results indicate that the 1,6-diyne compound is converted into the mitochondrial probe benzindole dimer compound in cells.

### Example 24 Use of PA-1 to PA-40 for detecting active oxygen (sodium hypochlorite, hydrogen peroxide, ozone, etc.)

The compound PA-1 is dissolved in dichloromethane and applied to five pieces of polytetrafluoroethylene carriers respectively, then sodium hypochlorite aqueous solutions with different concentrations (8% mass concentration of sodium hypochlorite aqueous solution, 50 times diluted solution, 500 times diluted solution, 500 times diluted solution, and NaClO-free aqueous solution) are dripped on the carriers. Then, heat at 100 °C for 30 seconds. The carrier will change from colorless to red, and under 365nm ultraviolet light, the fluorescence will gradually change from no fluorescence to strong fluorescence.

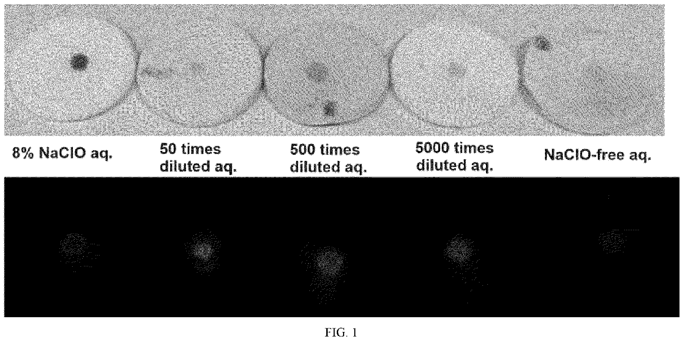

A typical test result is shown in FIG. 1, which shows the color development of compound PA-1 under fluorescence.

### Example 25 Fluorescence Performance Test of Benzisoindole Dimer Compounds

The fluorescence performance of the benzisoindole dimer compound is tested, with the specific testing method as follows: accurately weigh 1 mg of the sample and dissolve it in 10 mL of tetrahydrofuran. First, the absorption spectrum of the compound is measured, and the maximum absorption wavelength is used to measure the emission spectrum of the compound. The maximum emission wavelength can be obtained through the emission spectrum, and the optimal excitation wavelength of the maximum emission wavelength test compound can be selected. By selecting the optimal excitation wavelength to excite the compound, the final emission spectrum can be obtained.

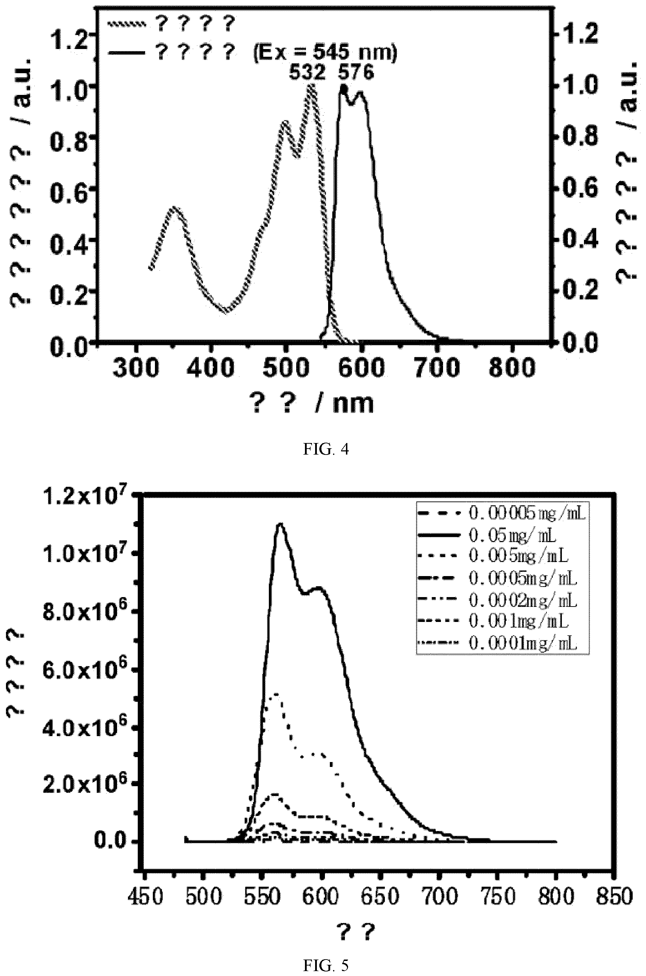

Typical test results are shown in FIG. 4, which presents the UV-visible absorption spectrum and emission spectrum of the compound FL-1 in the present application.

FIG. 5 shows the fluorescence emission spectrum of the compound FL-1 in the present application, illustrating the relationship between concentration and luminescence intensity.

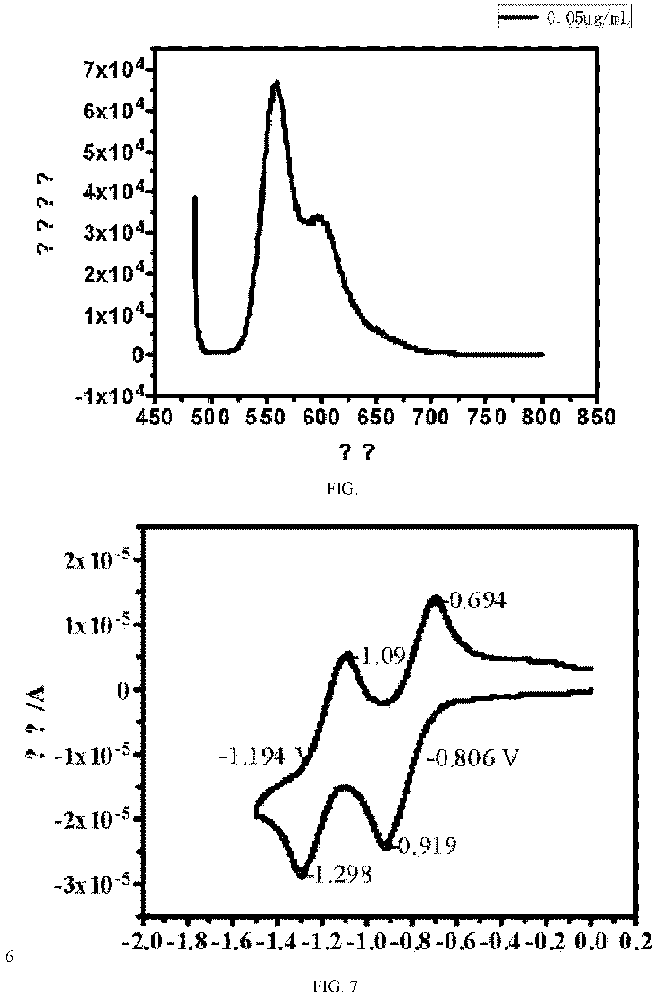

FIG. 6 shows the fluorescence emission spectrum of the compound FL-1 in the present application at a concentration of 0.05 micrograms per milliliter.

This indicates that s this type of compound has potential applications in fluorescence materials, fluorescence probes, etc.

### Example 26 Electrical Properties Test of Benzisoindole Dimer Compounds

The electrical properties of the benzisoindole dimer compounds are tested, with the specific testing method as follows: tetrabutylammonium hexafluorophosphate is used as the electrolyte at a concentration of 0.1M, and 5 mL of tetrahydrofuran is used as the solvent. The glassy carbon electrode is used as the working electrode, the platinum wire electrode is used as the auxiliary electrode, and the saturated calomel electrode is used as the reference electrode, with a scan rate of 100 mV/s.

The typical test result is shown in FIG. 7, which presents the cyclic voltammogram of the compound FL-1 in this application.

This indicates that this type of compound has two reduced states and may be used as conductive organic compounds or photo-redox catalysts.

The above are only a few Examples of the present application and do not constitute any form of limitation to the present application. Although the present application is disclosed as above with preferred embodiments, it is not intended to limit the present application. The person skilled in the art, without departing from the scope of the technical solution of the present application, using the technical content disclosed above to make slight changes or modifications are equivalent to equivalent implementation cases and fall within the scope of the technical solution.

## Claims

1. A compound represented by a general formula (I), and its tautomers, polymorphs, solvates, or salts thereof, 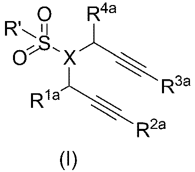
wherein, X is selected from nitrogen atom, phosphorus atom, arsenic atom, tellurium atom, and boron atom;
R' is selected from C₁-C₃₀ alkyl, substituted C₁-C₃₀ alkyl, C₆-C₃₀ aryl, substituted C₆-C₃₀ aryl, C₃-C₃₀ heteroaryl, substituted C₃-C₃₀ heteroaryl, amino, substituted amino and optionally inserted at any position by heteroatom groups selected from the following: CO, O, S, SO, SO₂, NR^{a}, -N=, =N-;
wherein, R^{1a}, R^{4a} are independently selected from hydrogen, deuterium, C₁-C₃₀ alkyl, substituted C₁-C₃₀ alkyl, C₆-C₃₀ aryl, substituted C₆-C₃₀ aryl, C₃-C₃₀ heteroaryl, substituted C₃-C₃₀ heteroaryl, phosphino, substituted phosphino, boryl, substituted boryl, silicon, substituted silicon, halogen, amino, substituted amino and optionally inserted at any position by heteroatom groups selected from the following: CO, O, S, SO, SO₂, NR^{a}, -N=, =N-;
R^{2a} is selected from C₆-C₃₀ aryl, substituted C₆-C₃₀ aryl, C₃-C₃₀ heteroaryl, substituted C₃-C₃₀ heteroaryl, C₁-C₁₀ alkenyl, C₁-C₁₀ substituted alkenyl;
R^{3a} is hydrogen, deuterium, C₁-C₃₀ alkyl, substituted C₁-C₃₀ alkyl, C₁-C₃₀ alkenyl, C₁-C₃₀ substituted alkenyl, C₁-C₃₀ alkynyl, substituted C₁-C₃₀ alkynyl, C₆-C₃₀ aryl, substituted C₆-C₃₀ aryl, C₃-C₃₀ heteroaryl, substituted C₃-C₃₀ heteroaryl, C₁-C₃₀ cycloalkyl, substituted C₁-C₃₀ cycloalkyl, C₁-C₃₀ heterocycloalkyl, substituted C₁-C₃₀ heterocycloalkyl, phosphino, halogen, silyl, boryl, germanium, arsenic, selenium and optionally inserted at any position by heteroatom groups selected from the following: CO, O, S, SO, SO₂, NR^{a}, -N=, =N-;
R^{a} is independently selected from H, alkyl or aryl.

2. The compound represented by the general formula (I) according to claim 1, wherein the substituents in the substituted C₆-C₃₀ aryl and the substituted C₃-C₃₀ heteroaryl are selected from alkyl, alkenyl, aldehyde, halogen, haloalkyl, ester-inserted alkyl, alkoxy, alkylthio, and substituted amino.

3. The compound represented by the general formula (I) according to claim 1, wherein the substituents in the substituted C₆-C₃₀ aryl and the substituted C₃-C₃₀ heteroaryl are selected from C₁-C₃₀ alkyl, C₁-C₃₀ alkenyl, C₁-C₃₀ aldehyde group, halogen, halogenated C₁-C₃₀ alkyl, ester-inserted C₁-C₃₀ alkyl, C₁-C₃₀ alkoxy, C₁-C₃₀ alkylthio, and phenyl-substituted amino.

4. The compound represented by the general formula (I) according to claim 1, wherein R' is selected from C₆-C₃₀ aryl, C₆-C₁₀ alkyl-substituted C₆-C₃₀ aryl, C₆-C₃₀ aryl substituted with "halogen substituted C₆-C₁₀ alkyl", C₃-C₃₀ heteroaryl, C₆-C₁₀ alkyl substituted C₃-C₃₀ heteroaryl, C₃-C₃₀ heteroaryl substituted with "halogen substituted C₆-C₁₀ alkyl"; R' is selected from C₆-C₃₀ aryl, C₃-C₃₀ heteroaryl.

5. The compound represented by the general formula (I) according to claim 1, wherein R^{1a}, R^{4a} are independently selected from hydrogen, aryl, alkyl-substituted C₆-C₃₀ aryl, C₆-C₃₀ aryl substituted with "S atom-inserted alkyl", C₆-C₃₀ aryl substituted with "O atom-inserted alkyl", halogen-substituted C₆-C₃₀ aryl, aryl-substituted C₆-C₃₀ aryl, C₆-C₃₀ aryl substituted with "halogen-substituted alkyl", C₆-C₃₀ aryl substituted with "aryl-substituted amino", C₆-C₃₀ aryl substituted with "ester-inserted alkyl", and C₃-C₃₀ heteroaryl.

6. The compound represented by the general formula (I) according to claim 1, wherein R^{1a} is independently selected from phenyl, tert-butyl substituted phenyl, CH₃S-substituted phenyl, CHaO-substituted phenyl, naphthyl, phenyl-substituted phenyl, phenyl substituted with at least one methyl, CF₃O-substituted phenyl, Br-substituted phenyl, phenyl substituted with "diphenyl-substituted amino", phenyl substituted with "ester -inserted methyl", dibenzothienyl.

7. The compound represented by the general formula (I) according to claim 1, wherein R^{2a} is selected from C₆-C₃₀ aryl, C₆-C₃₀ aryl substituted with "ester-inserted alkyl", alkyl-substituted C₆-C₃₀ aryl, C₆-C₃₀ aryl substituted with "oxygen atom-inserted alkyl", aryl-substituted C₆-C₃₀ aryl, C₃-C₃₀ heteroaryl, aldehyde-substituted C₆-C₃₀ aryl, halogen-substituted C₆-C₃₀ aryl, and alkenyl-substituted C₆-C₃₀ aryl.

8. The compound represented by the general formula (I) according to claim 1, wherein R^{2a} is selected from phenyl, naphthyl, phenyl substituted with "ester-inserted methyl", propyl-substituted phenyl, phenanthrenyl, phenyl substituted with "oxygen atom-inserted methyl", biphenyl, thienyl, formaldehyde-substituted phenyl, Cl-substituted phenyl, vinyl-substituted phenyl.

9. The compound represented by the general formula (I) according to claim 1, wherein R^{3a} is selected from hydrogen atom, deuterium atom, C₆-C₃₀ aryl, C₆-C₃₀ aryl substituted with "ester-inserted alkyl", C₆-C₃₀ aryl substituted with at least one alkyl.

10. The compound represented by the general formula (I) according to claim 1, wherein R^{3a} is selected from a hydrogen atom, a deuterium atom, phenyl, phenyl substituted with "ester-inserted methyl ", and phenyl substituted with at least one methyl.

11. The compound represented by formula (I) according to claim 1, wherein the tautomers have the general formula (II), the general formula (III) or the general formula (IV):

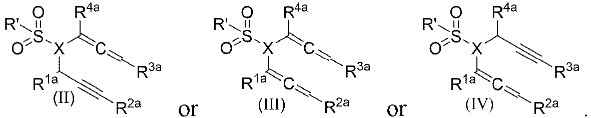

12. The compound represented by the general formula (I) according to claim 1, wherein the compound represented by formula (I) is selected from at least one of the following compounds:

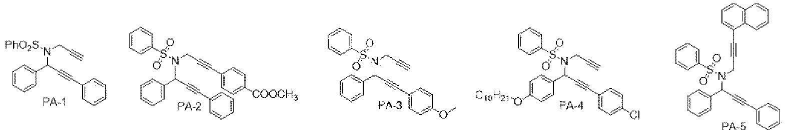

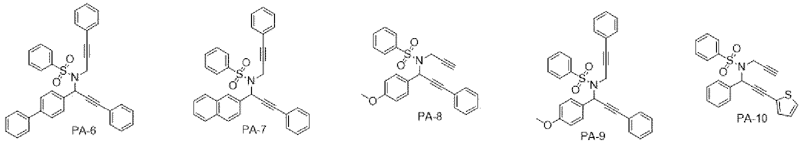

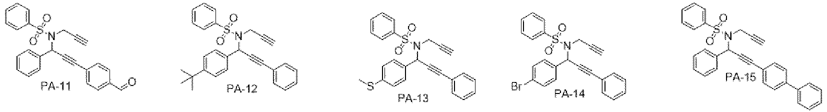

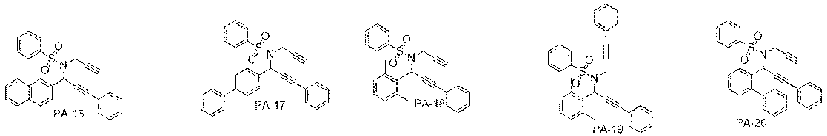

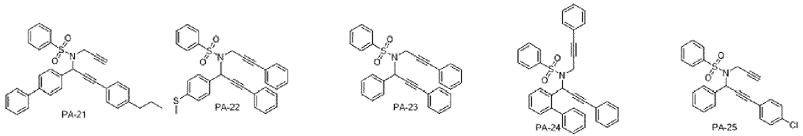

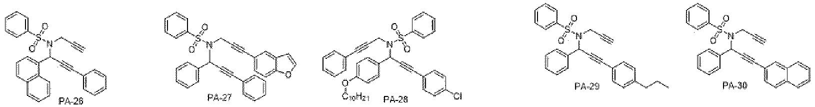

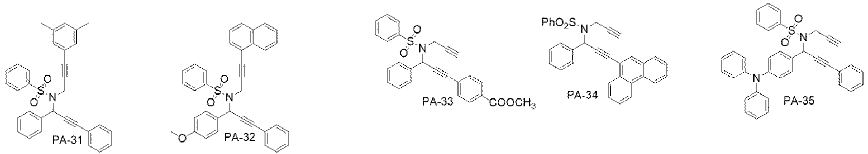

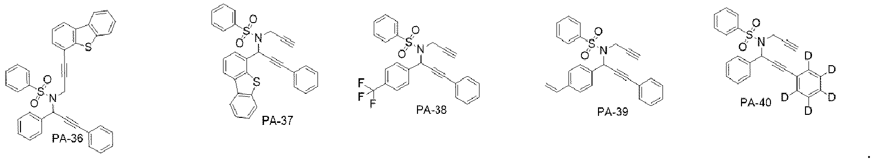

13. A preparation method of the compound according to any one of claims 1 to 12 containing the following steps:
reacting a raw material containing the compounds represented by the general formulas (V) and the general formula (VI) to obtain the compound represented by the general formula (I);

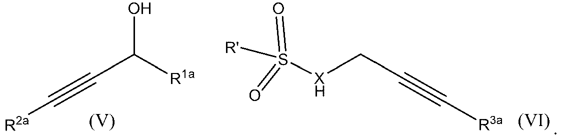

14. The preparation method according to claim 13, wherein a molar ratio of the compounds represented by the general formula (V) and the general formula (VI) is in a range from 1: 1 to 1:5.

15. The preparation method according to claim 13, wherein the reaction is carried out in the presence of an acid reagent; the acid reagent includes p-toluenesulfonic acid, phenylsulfonic acid, p-nitrobenzenesulfonic acid, methanesulfonic acid, ferric chloride, or aluminum trichloride.

16. The preparation method according to claim 13, wherein the reaction temperature is in a range from 25°C to 100°C; the reaction time is in a range from 0.1 h to 48 h.

17. The preparation method according to claim 13, wherein the preparation method of the compound represented by the general formula (V) includes the following steps:
reacting a raw material containing the compounds represented by the general formulas (VII) and the general formula (VIII) to obtain the compound represented by the general formula (V);

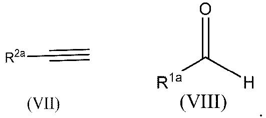

18. The preparation method according to claim 17, wherein a molar ratio of the compounds represented by general formula (VII) and (VIII) is in a range from 1: 1 to 1:5.

19. The preparation method according to claim 17, wherein the reaction is carried out in the presence of a nucleophilic substitution reagent; the nucleophilic substitution reagent includes n-butyllithium, sec-butyllithium, tert-butyllithium, methyllithium, diisopropylaminolithium, and bis(trimethylsilyl)aminolithium.

20. The preparation method according to claim 17, wherein the reaction temperature is in a range from -78°C to 50°C; the reaction time is in a range from 0.1h to 24h.

21. A method for preparing a 3-arylbenzisoindole compound, containing the following steps:
reacting a mixture containing a 1,6-diyne compound, a base and an organic solvent I to obtain the 3-arylbenzisoindole compound;
the 1,6-diyne compound is selected from the compounds represented by the general formula (I) described in any one of claims 1 to 20.

22. The preparation method according to claim 21, wherein the base is at least one selected from cesium carbonate, potassium carbonate and sodium carbonate.

23. The preparation method according to claim 21, wherein the organic solvent I is at least one selected from methanol, isopropyl alcohol, n-butanol, toluene and xylene.

24. The preparation method according to claim 21, wherein a molar ratio of the 1,6-diyne compound to the base is in a range from 1:0.1 to 1:10; a concentration of the 1,6-diyne compound is in a range from 20mM to 1M.

25. The preparation method according to claim 21, wherein a molar ratio of the 1,6-diyne compound to the base is in a range from 1:1 to 1:10.

26. The preparation method according to claim 21, wherein conditions for the reaction III are as follows:
reaction temperature is in a range from 25°C to 150°C;
reaction time is in a range from 0.5h to 72h.

27. The preparation method according to claim 21, wherein the reaction atmosphere is an inert gas.

28. A 3-arylbenzoisoindole compound obtained by the preparation method according to any one of claims 21 to 27 and its tautomers, polymorphs, solvates, or salts thereof, wherein the 3-arylbenzisoindole compound has the structural formula described in Formula A: 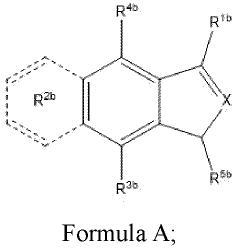
wherein X is one selected from nitrogen atom, phosphorus atom, arsenic atom, tellurium atom, and boron atom;
R^{1b} and R^{5b} have the same scope as R^{1a} in claim 1; R^{3b} and R^{4b} have the same scope as R^{1a} in claim 1; R^{2b} is selected from C₆-C₃₀ aryl, substituted C₆-C₃₀ aryl, C₃-C₃₀ heteroaryl, substituted C₃-C₃₀ heteroaryl.

29. A method for preparing a benzisoindole dimer compound, containing the following steps:
reacting a mixture containing a 3-aryl benzoisoindole compound, an oxidant, and an organic solvent II to obtain the benzoisoindole dimer compound;
the 3-aryl benzoisoindole compound is selected from the 3-aryl benzoisoindole compound according to claim 28 and its tautomeric isomers, polymorphs, solvates, or salts thereof.

30. The preparation method according to claim 29, wherein the oxidant is at least one selected from 2,2,6,6-tetramethylpiperidine nitrogen oxide, oxygen, sodium hypochlorite, aqueous hydrogen peroxide solution, tert-butanol peroxide, lauroyl peroxide, benzoyl peroxide, tert-butyl perbenzoate, m-chloroperoxybenzoic acid, peracetic acid, and di-tert-butyl peroxide.

31. The preparation method according to claim 29, wherein the organic solvent II is at least one selected from methanol, ethanol, isopropanol, p-xylene, n-butanol, ethyl acetate, acetonitrile, N,N-dimethylformamide and dimethyl sulfoxide.

32. The preparation method according to claim 29, wherein conditions for reaction II are as follows:
reaction temperature is in a range from 25°C to 150°C;
reaction time is in a range from 0.5h to 72h.

33. A benzisoindole dimer compound obtained by the preparation method according to any one of claims 29 to 31, wherein the benzisoindole dimer compound has the structure shown in the following formula; 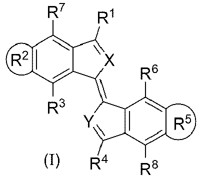
wherein, X and Y are independently selected from nitrogen atom, phosphorus atom, arsenic atom, tellurium atom, and boron atom;
R¹ and R⁴ are the same or different; R¹ and R⁴ have the same scope as R^{1a} in claim 1;
R² and R⁵ are the same or different; R² and R⁵ have the same scope as R^{2b} in claim 7;
R³ and R⁶ are the same or different; R⁷ and R⁸ are the same or different; R³, R⁶, R⁷ and R⁸ have the same scope as R^{1a} in claim 1.

34. The benzisoindole dimer compound according to claim 33, wherein it has a structure shown in formula (II); 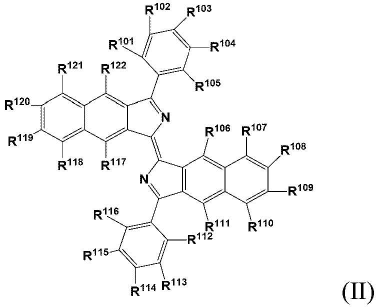
wherein R¹⁰¹, R¹⁰², R¹⁰³, R¹⁰⁴, R¹⁰⁵, R¹⁰⁶, R¹⁰⁷, R¹⁰⁸, R¹⁰⁹, R¹¹⁰, R¹¹¹, R¹¹², R¹¹³, R¹¹⁴, R¹¹⁵, R¹¹⁶, R¹¹⁷, R¹¹⁸, R¹¹⁹, R¹²⁰, R¹²¹, R¹²² are independently selected from hydrogen atom, C₁-C₁₅ alkyl, substituted C₁-C₁₅ alkyl, C₁-C₁₅ alkenyl, substituted C₁-C₁₅ alkenyl, C₁-C₁₅ alkynyl, substituted C₁-C₁₅ alkynyl, C₆-C₃₀ aryl, substituted C₆-C₃₀ aryl, C₃-C₃₀ heteroaryl, substituted C₃-C₃₀ heteroaryl, aldehyde, phosphine, halogen, silyl, boron, germanium, arsenic, selenium and optionally inserted at any position by a heteroatom group selected from the following: CO, O, S, SO, SO₂, N, NR^{a}, -N=, =N-; any two adjacent groups among R¹⁰¹, R¹⁰², R¹⁰³, R¹⁰⁴, R¹⁰⁵, R¹⁰⁶, R¹⁰⁷, R¹⁰⁸, R¹⁰⁹, R¹¹⁰, R¹¹¹, R¹¹², R¹¹³, R¹¹⁴, R¹¹⁵, R¹¹⁶, R¹¹⁷, R¹¹⁸, R¹¹⁹, R¹²⁰, R¹²¹, R¹²² can form a ring;
R^{a} is independently selected from H, alkyl or aryl.

35. The benzisoindole dimer compound according to claim 34, wherein R¹⁰¹, R¹⁰², R¹⁰³, R¹⁰⁴, R¹⁰⁵, R¹⁰⁶, R¹⁰⁷, R¹⁰⁸, R¹⁰⁹, R¹¹⁰, R¹¹¹, R¹¹², R¹¹³, R¹¹⁴, R¹¹⁵, R¹¹⁶, R¹¹⁷, R¹¹⁸, R¹¹⁹, R¹²⁰, R¹²¹, R¹²² are independently selected from hydrogen atom, C₁-C₁₅ alkyl, halogen-substituted C₁-C₁₅ alkyl, aryl-substituted C₁-C₁₅ alkyl, S-inserted C₁-C₁₅ alkyl, O-inserted C₁-C₁₅ alkyl, ester-inserted C₁-C₁₅ alkyl, C₁-C₁₅ alkenyl, C₆-C₃₀ aryl, alkyl-substituted C₆-C₃₀ aryl, C₃-C₃₀ heteroaryl, substituted C₃-C₃₀ heteroaryl, and optionally inserted at any position by the following heteroatom groups: CO, O, S, SO, SO₂, N, NR^{a}, -N=, =N-; any two adjacent groups among R¹⁰¹, R¹⁰², R¹⁰³, R¹⁰⁴, R¹⁰⁵, R¹⁰⁶, R¹⁰⁷, R¹⁰⁸, R¹⁰⁹, R¹¹⁰, R¹¹¹, R¹¹², R¹¹³, R¹¹⁴, R¹¹⁵, R¹¹⁶, R¹¹⁷, R¹¹⁸, R¹¹⁹, R¹²⁰, R¹²¹, R¹²² can form a ring;
R^{a} is independently selected from H, alkyl or aryl.

36. The benzisoindole dimer compound according to claim 33, wherein it has the structure shown in formula (III); 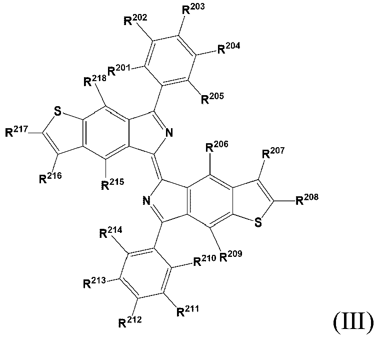
wherein R²⁰¹, R²⁰², R²⁰³, R²⁰⁴, R²⁰⁵, R²⁰⁶, R²⁰⁷, R²⁰⁸, R²⁰⁹, R²¹⁰, R²¹¹, R²¹², R²¹³, R²¹⁴, R²¹⁵, R²¹⁶, R²¹⁷, and R²¹⁸ are independently selected from hydrogen atom, C₁-C₁₅ alkyl, substituted C₁-C₁₅ alkyl, C₁-C₁₅ alkenyl, substituted C₁-C₁₅ alkenyl, C₁-C₁₅ alkynyl, substituted C₁-C₁₅ alkynyl, C₆-C₃₀ aryl, substituted C₆-C₃₀ aryl, C₃-C₃₀ heteroaryl, substituted C₃-C₃₀ heteroaryl, aldehyde, phosphine, halogen, silyl, boron, germanium, arsenic, selenium and optionally inserted at any position by a heteroatom group selected from the following: CO, O, S, SO, SO₂, N, NR^{a}, -N=, =N-; any two adjacent groups among R²⁰¹, R²⁰², R²⁰³, R²⁰⁴, R²⁰⁵, R²⁰⁶, R²⁰⁷, R²⁰⁸, R²⁰⁹, R²¹⁰, R²¹¹, R²¹², R²¹³, R²¹⁴, R²¹⁵, R²¹⁶, R²¹⁷, R²¹⁸ can form a ring;
R^{a} is independently selected from H, alkyl or aryl.

37. The benzisoindole dimer compound according to claim 36, wherein R²⁰¹, R²⁰², R²⁰³, R²⁰⁴, R²⁰⁵, R²⁰⁶, R²⁰⁷, R²⁰⁸, R²⁰⁹, R²¹⁰, R²¹¹, R²¹², R²¹³, R²¹⁴, R²¹⁵, R²¹⁶, R²¹⁷, and R²¹⁸ are independently selected from hydrogen atom, C₁-C₁₅ alkyl, halogen-substituted C₁-C₁₅ alkyl, aryl-substituted C₁-C₁₅ alkyl, S-inserted C₁-C₁₅ alkyl, O-inserted C₁-C₁₅ alkyl, ester-inserted C₁-C₁₅ alkyl, C₁-C₁₅ alkenyl, C₆-C₃₀ aryl, alkyl-substituted C₆-C₃₀ aryl, C₃-C₃₀ heteroaryl, substituted C₃-C₃₀ heteroaryl, and optionally inserted at any position by the following heteroatom groups: CO, O, S, SO, SO₂, N, NR^{a}, -N=, =N-; any two adjacent groups among R²⁰¹, R²⁰², R²⁰³, R²⁰⁴, R²⁰⁵, R²⁰⁶, R²⁰⁷, R²⁰⁸, R²⁰⁹, R²¹⁰, R²¹¹, R²¹², R²¹³, R²¹⁴, R²¹⁵, R²¹⁶, R²¹⁷, and R²¹⁸ can form a ring;
R^{a} is independently selected from H, alkyl or aryl.

38. Use of the compound represented by the general formula (I) according to any one of claims 1 to 12 and/or the compound obtained by the preparation method according to any one of claims 13 to 20 in the preparation of mitochondrial fluorescent probes, in the detection of substances containing active oxygen and in cell imaging.

39. The use according to claim 38, wherein the method for preparing the mitochondrial fluorescent probe contains the following steps:
incubating the culture dish containing the 1,6-diyne compound and mouse fibroblasts.

40. The use according to claim 39, wherein volume of a solution of the 1,6-diyne compound is in a range from 1 µL to 1000 µL.

41. The use according to claim 39, wherein concentration of a solution of the 1,6-diyne compound is in a range from 1 µM to 1000 µM.

42. The use according to claim 39, wherein conditions for incubation are as follows:
reaction temperature is in a range from 5 °C to 50°C; reaction time is in a range from 0.1h to 48h.

43. The use according to claim 38, wherein the compound represented by the general formula (I) is used in preparation of mitochondrial fluorescent probes of mouse fibroblasts.

44. The use according to claim 38, wherein the compound represented by the general formula (I) is used in preparation of mitochondrial fluorescent probes for mouse mononuclear macrophage leukemia cells or HER2-breast cancer overexpression cells or human liver cancer cell lines.

45. The use according to claim 38, wherein the active oxygen includes at least one of sodium hypochlorite, hydrogen peroxide, and ozone.

46. Use of the 3-arylbenzoisoindole compound obtained by the preparation method according to any one of claims 21 to 27 and/or the 3-arylbenzoisoindole compound according to claim 28 and its tautomers, polymorphs, solvates, or their salts in lysosomal fluorescent probe, autophagy lysosome fluorescent probe, synthesis of benzisoindole dimer compound, optical super-resolution microscopy, confocal microscopy, wide-field microscopy, fluorescence lifetime imaging microscopy, fluorescence resonance energy transfer microscopy, super-resolution optical wave imaging, fluorescence activation loclization microscopy, and light-emitting device.

47. Use of the benzisoindole dimer compound obtained by the preparation method according to any one of claims 29 to 32 and/or the benzisoindole dimer compound according to any one of claims 33 to 37 in mitochondrial fluorescence probes, optical super-resolution microscopy, confocal microscopy, wide-field microscopy, fluorescence lifetime imaging microscopy, fluorescence resonance energy transfer microscopy, super-resolution optical fluctuation imaging, fluorescence photoactivated localization microscopy, light-emitting devices, cellular imaging, and fluorescent dyes.

48. The use according to claim 47, wherein the benzisoindole dimer compound is produced by the compound represented by the general formula (I) according to any one of claims 1 to 12 during cell incubation.

49. The use according to claim 48, wherein excitation wavelength of the benzisoindole dimer compound is in a range from 220 nm to 1000 nm.

50. The use according to claim 48, wherein emission wavelength of the benzisoindole dimer compound is in a range from 400 nm to 800 nm.
